# EUROPÄISCHE PATENTSCHRIFT

(11) **EP 1 973 641 B1**
(45) Veröffentlichungstag und Bekanntmachung des Hinweises auf die Patenterteilung: **22.07.2009**
(21) Anmeldenummer: 06841539.7
(22) Anmeldetag: 21.12.2006
(51) Int. Cl.: B01J 8/06, C07C 45/35, C07C 51/25

(54) **VERFAHREN DER HETEROGEN KATALYSIERTEN GASPHASEN-PARTIALOXIDATION WENIGSTENS EINER ORGANISCHEN AUSGANGSVERBINDUNG**
PROCESS FOR HETEROGENEOUSLY CATALYSED GAS PHASE PARTIAL OXIDATION OF AT LEAST ONE ORGANIC STARTING COMPOUND
PROCEDE D'OXYDATION PARTIELLE EN PHASE GAZEUSE, PAR CATALYSE HETEROGENE, D'AU MOINS UN COMPOSE ORGANIQUE DE DEPART

(30) Priorität: 05.01.2006 DE 102006000996; 05.01.2006 US 756207 P
(43) Veröffentlichungstag der Anmeldung: 01.10.2008
(73) Patentinhaber: BASF SE, 67056 Ludwigshafen (DE)
(72) Erfinder: CREMER, Ulrich, 68161 Mannheim (DE); WEGERLE, Ulrike, 67550 Worms (DE); LAQUA, Gerhard, 67112 Mutterstadt (DE); HAMMON, Ulrich, 68163 Mannheim (DE); MÜLLER-ENGEL, Klaus Joachim, 76297 Stutensee (DE)
(86) Internationale Anmeldenummer: PCT/EP2006/070069
(87) Internationale Veröffentlichungsnummer: WO 2007/077145

(56) Entgegenhaltungen:
- WO-A-20/04009525
- DE-A1-5102004 025 44

## Beschreibung

Vorliegende Erfindung betrifft ein Verfahren der heterogen katalysierten Gasphasen-Partialoxidation wenigstens einer organischen Ausgangsverbindung mit molekularem Sauerstoff an einem in einem Reaktionsraum frisch eingebrachten Katalysatorfestbett, bei dem man zum Zweck der Partialoxidation ein die wenigstens eine organische Ausgangsverbindung und den molekularen Sauerstoff enthaltendes Reaktionsgasgemisch durch das Katalysatorfestbett führt sowie durch indirekten Wärmeaustausch mit einem außerhalb des Reaktionsraums geführten fluiden Wärmeträger Reaktionswärme abführt und dann, wenn mit zunehmender Betriebsdauer eine zunehmende Minderung der Qualität des Katalysatorfestbetts einhergeht, zur Rückgewinnung der Qualität des Katalysatorfestbetts nicht das gesamte Katalysatorfestbett sondern nur einen Teil desselben durch ein Ersatzkatalysatorteilfestbett ersetzt.

Unter einer vollständigen Oxidation einer organischen Verbindung mit molekularem Sauerstoff wird hier verstanden, dass die organische Verbindung unter reaktiver Einwirkung von molekularem Sauerstoff so umgesetzt wird, dass der in der organischen Verbindung insgesamt enthaltene Kohlenstoff in Oxide des Kohlenstoffs und der in der organischen Verbindung insgesamt enthaltene Wasserstoff in Oxide des Wasserstoffs umgewandelt wird. Alle davon verschiedenen Umsetzungen einer organischen Verbindung unter reaktiver Einwirkung von molekularem Sauerstoff werden hier als Partialoxidationen einer organischen Verbindung zusammengefasst.

Im besonderen sollen hier unter Partialoxidationen solche Umsetzungen organischer Verbindungen unter reaktiver Einwirkung von molekularem Sauerstoff verstanden werden, bei denen die partielle zu oxidierende organische Verbindung nach beendeter Umsetzung wenigstens ein Sauerstoffatom mehr chemisch gebunden enthält als vor Durchführung der Partialoxidation.

Als ein sich unter den Bedingungen der heterogen katalysierten Gasphasen-Partialoxidation im wesentlichen inert verhaltendes Verdünnungsgas werden solche Verdünnungsgase verstanden, deren Bestandteile unter den Bedingungen der heterogen katalysierten Gasphasen-Partialoxidation - jeder Bestandteil für sich betrachtet - zu mehr als 95 mol-%, vorzugsweise zu mehr als 99 mol-% unverändert erhalten bleiben.

Unter der Belastung eines einen Reaktionsschritt katalysierenden Katalysatorfestbetts mit Reaktionsgasgemisch wird die Menge an Reaktionsgasgemisch in Normlitern (= NI; das Volumen in Litern, das die entsprechende Reaktionsgasgemischmenge bei Normalbedingungen, d. h., bei 25 °C und 1 atm, einnehmen würde) verstanden, die dem Katalysatorfestbett, bezogen auf das Volumen seiner Schüttung (reine Inertmaterialabschnitte werden dabei nicht miteinbezogen), pro Stunde zugeführt wird (→ Einheit = Nl/l·h). Die Belastung kann auch nur auf einen Bestandteil des Reaktionsgasgemischs bezogen sein. Dann ist es die Volumenmenge dieses Bestandteils, die dem Katalysatorfestbett, bezogen auf das Volumen seiner Schüttung, pro Stunde zugeführt wird.

Es ist allgemein bekannt, dass durch partielle und heterogen katalysierte Oxidation verschiedenster organischer Ausgangsverbindungen mit molekularem Sauerstoff in der Gasphase im Katalysatorfestbett zahlreiche Grundchemikalien (Zielprodukte) erzeugt werden können. Beispielhaft genannt seien die Umsetzung von Propylen zu Acrolein und/oder Acrylsäure (vgl. z. B. DE-A 23 51 151), die Umsetzung von tert.-Butanol, iso-Buten, iso-Butan, iso-Butyraldehyd oder dem Methylether des tert.-Butanols zu Metacrolein und/oder Methacrylsäure (vgl. z. B. DE-A 25 26 238, EP-A 092 097, EP-A 058 927, DE-A 41 32 263, DE-A 41 32 684 und DE-A 40 22 212), die Umsetzung von Acrolein zu Acrylsäure, die Umsetzung von Methacrolein zu Methacrylsäure (vgl. z. B. die DE-A 25 26 238), die Umsetzung von o-Xylol, p-Xylol oder Naphtalin zu Phthalsäureanhydrid (vgl. z. B. EP-A 522 871) oder den entsprechenden Säuren sowie die Umsetzung von Butadien zu Maleinsäureanhydrid (vgl. z. B. GB-A 1 464 198 und GB-A 1 291 354), die Umsetzung von Indanen zu z. B. Anthrachinon (vgl. z. B. DE-A 20 25 430), die Umsetzung von Ethylen zu Ethylenoxid oder von Propylen zu Propylenoxid (vgl. z. B. DE-AS 12 54 137, DE-A 21 59 346, EP-A 372 972, WO 89/07101, DE-A 43 11 608 und Beyer, Lehrbuch der organischen Chemie, 17. Auflage (1973), Hirzel Verlag Stuttgart, Seite 261), die Umsetzung von Propylen und/oder Acrolein zu Acrylnitril (vgl. z. B. die DE-A 23 51 151), die Umsetzung von iso-Buten und/oder Methacrolein zu Methacrylnitril (d. h., der Begriff der partiellen Oxidation soll in dieser Schrift auch die partielle Ammoxidation, d. h., eine partielle Oxidation im Beisein von Ammoniak umfassen), die oxidative Dehydrierung von Kohlenwasserstoffen (vgl. z. B. die DE-A 23 51 151), die Umsetzung von Propan zu Acrylnitril oder zu Acrolein und/oder Acrylsäure (vgl. z. B. DE-A 101 31 297, EP-A 1 090 684, EP-A 608 838, DE-A 100 46 672, EP-A 529 853, WO 01/96270 und DE-A 100 28 582), die Umsetzung von iso-Butan zu Methacrolein und/oder Methacrylsäure, sowie die Reaktionen von Ethan zu Essigsäure, von Ethylen zu Ethylenoxid, von Benzol zu Phenol sowie von 1-Buten oder 2-Buten zu den entsprechenden Butandiolen etc..

Dem Katalysatorfestbett kommt dabei die Aufgabe zu, zu bewirken, dass die gewünschte Gasphasen-Partialoxidation gegenüber der vollständigen Oxidation bevorzugt abläuft.

Die chemische Umsetzung erfolgt, wenn das Reaktionsgasgemisch das Festbett durchströmt während der Verweilzeit des Reaktionsgasgemisches in selbigem.

Bei den Festkörperkatalysatoren handelt es sich häufig um Oxidmassen oder um Edelmetalle (z. B. Ag). Die katalytisch aktive Oxidmasse kann neben Sauerstoff lediglich ein anderes Element oder mehr als ein anderes Element (im Fall von sogenannten Multielementoxidmassen) enthalten.

Besonders häufig kommen als katalytisch aktive Oxidmassen solche zur Anwendung, die mehr als eine metallisches, insbesondere übergangsmetallisches, Element umfassen. In diesem Fall spricht man von Multimetalloxidmassen. Üblicherweise sind diese keine einfachen physikalischen Gemische von Oxiden ihrer elementaren Konstituenten, sondern Gemische von komplexen Polyverbindungen dieser Elemente. In der Praxis werden die vorgenannten katalytisch aktiven Festmassen in der Regel zu unterschiedlichsten Geometrien geformt (Ringe, Vollzylinder, Kugeln etc.) eingesetzt. Die Formgebung (zum Formkörper) kann dabei so erfolgen, dass die katalytisch aktive Masse als solche (z. B. in Extrudern oder Tablettiervorrichtungen) geformt wird, so dass im Ergebnis ein sogenannter Vollkatalysator resultiert, oder dadurch, dass die Aktivmasse auf einen vorgeformten Träger aufgebracht wird (vgl. z. B. WO 2004/009525 und WO 2005/113127).

Beispiele für Katalysatoren die für erfindungsgemäße heterogen katalysierte Festbett-Gasphasen-Partialoxidationen wenigstens einer organischen Ausgangsverbindung geeignet sind, finden sich z. B. in der DE-A 100 46 957, in der EP-A 1 097 745, in der DE-A 44 31 957, in der DE-A 100 46 928, in der DE-A 199 10 506, in der DE-A 196 22 331, in der DE-A 101 21 592, in der EP-A 700 714, in der DE-A 199 10 508, in der EP-A 415 347, in der EP-A 471 853 und in der EP-A 700 893).

Üblicherweise verlaufen heterogen katalysierte Gasphasen-Partialoxidationen stark exotherm. Infolge einer Vielfalt möglicher Parallel- und/oder Folgereaktionen ist im Hinblick auf eine möglichst selektive Umsetzung der partiell zu oxidierenden wenigstens einen organischen Ausgangsverbindung zum gewünschten Zielprodukt die alleinige Maßnahme einer Katalysatormitverwendung normalerweise nicht ausreichend. Vielmehr ist es für eine möglichst selektive Durchführung einer heterogen katalysierten Gasphasen-Partialoxidation im Katalysatorfestbett zusätzlich erforderlich, den Verlauf der Reaktionstemperatur bzw. den Verlauf der Temperatur des Katalysatorfestbetts in Strömungsrichtung des Reaktionsgasgemischs in einem gewissen Umfang zu lenkern. Aus Gründen der Wärmeabfuhr führt man derartige Partialoxidationen daher in der Regel in "isothermen" Festbettreaktoren durch, bei denen sich das Katalysatorfestbett in einen Reaktionsraum eingebracht befindet, um den zum Zweck des indirekten Wärmeaustauschs außerhalb des Reaktionsraums ein fluider Wärmeträger (ein Wärmeaustauschmittel) geführt wird, der die materielle Einhüllende des Reaktionsraums (die Wand des Reaktionsraums) berührt (sich mit selbiger in Kontakt befindet). Beispielsweise kann sich das Katalysatorfestbett in den Kontaktrohren eines Rohrbündelreaktors befinden, um die zur Wärmeabfuhr eine Salz- oder einer Metallschmelze geführt (geleitet) wird.

Zusätzlich werden die Reaktionspartner üblicherweise mit einem sich unter den Bedingungen der heterogen katalysierten Gasphasen-Partialoxidation im wesentlichen inerten Gas verdünnt, das mit seiner Wärmekapazität frei werdende Reaktionswärme zu absorbieren vermag.

Das Reaktionsgasgemisch einer heterogen katalysierten Gasphasen-Partialoxidation wenigstens einer organischen Ausgangsverbindung wird daher neben der wenigstens einen organischen Ausgangsverbindung und molekularem Sauerstoff in der Regel zusätzlich wenigstens ein inertes Verdünnungsgas umfassen.

Eines der häufigsten mitverwendeten inerten Verdünnungsgase ist molekularer Stickstoff, der automatisch immer dann zur Anwendung kommt, wenn als Sauerstoffquelle für die heterogen katalysierte Gasphasen-Partialoxidation Luft verwendet wird.

Ein anderes vielfach mitverwendetes inertes Verdünnungsgas ist wegen seiner allgemeinen Verfügbarkeit Wasserdampf.

Andere in typischer Weise mitverwendete inerte Verdünnungsgase sind Edelgase (z. B. He, Ar, Ne) oder die Kohlenoxide CO₂ und/oder CO.

Die Verwendung von Verdünnungsgasen mit möglichst großer molarer Wärmekapazität ist üblicherweise besonders vorteilhaft (vgl. z. B. EP-A 253 409). Dazu zählen z. B. im Fall einer Partialoxidation einer ungesättigten organischen Ausgangsverbindung u. a. häufig gesättigte Kohlenwasserstoffe wie z. B. Propan im Fall einer Partialoxidation von Propylen zu Acrolein und/oder Acrylsäure.

Vielfach wird auch Kreisgas als inertes Verdünnungsgas mitverwendet (vgl. z. B. EP-A 1 180 508). Als Kreisgas wird das Restgas bezeichnet, das nach einer einstufigen oder mehrstufigen (bei der mehrstufigen heterogen katalysierten Gasphasen-Partialoxidation wenigstens einer organischen Ausgangsverbindung wird die Gasphasen-Partialoxidation im Unterschied zur einstufigen heterogen katalysierten Gasphasen-Partialoxidation nicht in einem sondern in wenigstens zwei hintereinandergeschalteten Reaktorabschnitten (Reaktionsräumen) durchgeführt (die in einem gemeinsamen Gehäuse nahtlos ineinander übergehen oder in zwei räumlich getrennten, hintereinander geschalteten, Reaktoren untergebracht sein können), wobei zwischen aufeinanderfolgenden Reaktorabschnitten bzw. Reaktoren gegebenenfalls Inertgas und/oder Oxidationsmittel ergänzt wird; die Mehrstufigkeit wird insbesondere dann angewandt, wenn sich die Partialoxidation in aufeinanderfolgenden Schritten vollzieht; in diesen Fällen ist es häufig zweckmäßig, sowohl das Katalysatorfestbett als auch die sonstigen Reaktionsbedingungen an den jeweiligen Reaktionsschritt optimierend anzupassen, und den Reaktionsschritt in einem eigenen Reaktorabschnitt bzw. in einem eigenen Reaktor, d. h., als eine bzw. in einer separaten Reaktionsstufe durchzuführen; sie kann aber auch dann angewandt werden, wenn aus Gründen der Wärmeabfuhr oder aus anderen Gründen (vgl. z. B. DE-A 199 02 562) der Umsatz auf mehrere hintereinandergeschaltete Reaktorabschnitte bzw. Reaktoren verschmiert wird; ein Beispiel für eine häufig zweistufig durchgeführte heterogen katalysierte Gasphasen-Partialoxidation ist die Partialoxidation von Propylen zu Acrylsäure; in der ersten Reaktionsstufe wird das Propylen zum Acrolein und in der zweiten Reaktionsstufe das Acrolein zur Acrylsäure partialoxidiert; in entsprechender Weise wird auch häufig die Methacrylsäureherstellung, meist ausgehend von iso-Buten, zweistufig durchgeführt; beide vorgenannten Partialoxidationen können bei Verwendung geeigneter Katalysatorbeschickungen aber auch einstufig (beide Schritte in einem Reaktorabschnitt an einem Katalysatorfestbett mit beide Schritte katalysierendem Katalysator) durchgeführt werden, wie es z. B. für die Partialoxidation von Propylen zu Acrylsäure in der DE-A 101 21 592 beschrieben ist) heterogen katalysierten Gasphasen-Partialoxidation wenigstens einer organischen Verbindung dann verbleibt, wenn man aus dem Produktgasgemisch das Zielprodukt mehr oder weniger selektiv (z. B. durch Absorption in ein geeignetes Lösungsmittel oder durch fraktionierende Kondensation) abgetrennt hat. Im Regelfall besteht es überwiegend aus den für die Partialoxidation verwendeten inerten Verdünnungsgasen sowie aus bei der Partialoxidation üblicherweise als Nebenprodukt gebildetem oder als Verdünnungsgas zugesetztem Wasserdampf und durch unerwünschte vollständige Oxidation gebildeten Kohlenoxiden. Teilweise enthält es noch geringe Mengen an bei der Partialoxidation nicht verbrauchtem molekularem Sauerstoff (Restsauerstoff) und/oder an nicht umgesetzter organischer Ausgangsverbindung.

Die mitverwendeten inerten Verdünnungsgase sind aber nicht nur dabei behilflich, die Reaktionswärme aufzunehmen, sondern gewährleisten in der Regel gleichzeitig einen sicheren Betrieb der heterogen katalysierten Gasphasen-Partialoxidation der wenigstens einen organischen Ausgangsverbindung, indem sie das Reaktionsgasgemisch entweder außerhalb des Explosionsbereichs oder in einer noch sicher beherrschbaren Region des explosiven Bereichs halten.

Trotz der beschriebenen externen und internen Maßnahmen zur Lenkung (Steuerung) der Reaktionstemperatur bzw. der Temperatur des Katalysatorfestbetts muss aufgrund ihrer normalerweise trotzdem gegebenen Verschiedenheit zwischen der "Temperatur des Katalysatorfestbetts" und der "effektiven Temperatur des Katalysatorfestbetts" unterschieden werden.

Unter der Temperatur des Katalysatorfestbetts wird dabei die Temperatur des Katalysatorfestbetts bei Ausübung des Partialoxidationsverfahrens, jedoch in fiktiver Abwesenheit einer chemischen Reaktion (d.h., ohne den Einfluss der Reaktionswärme) verstanden (d.h., der Einfluss des außerhalb des Reaktionsraums geführten fluiden Wärmeträgers ist in gleicher Weise wie bei Ausübung des Partialoxidationsverfahrens einbezogen). Unter effektiver Temperatur des Katalysatorfestbetts wird dagegen die tatsächliche Temperatur des Katalysatorfestbetts unter zusätzlichem Einbezug der Partialoxidation verstanden. Ist die Temperatur eines Katalysatorfestbetts längs des Katalysatorfestbetts nicht konstant (z.B. im Fall von mehreren Temperaturzonen), so meint der Begriff Temperatur des Katalysatorfestbetts in dieser Schrift den (Zahlen) Mittelwert der Temperatur entlang des Katalysatorfestbetts. Das erfindungsgemäße Verfahren eignet sich jedoch insbesondere dann, wenn die Temperatur des Katalysatorfestbetts in Strömungsrichtung des Reaktionsgasgemischs längs des Katalysatorfestbetts konstant ist.

Von Bedeutung im vorgenannten Zusammenhang ist, dass die Temperatur des Reaktionsgasgemischs (und damit auch die effektive Temperatur des Katalysatorfestbetts) beim Durchschreiten des Katalysatorfestbetts in Strömungsrichtung des Reaktionsgasgemischs in der jeweiligen Reaktionsstufe üblicherweise einen Höchstwert (den sogenannten Heißpunktwert) durchläuft. Die Differenz zwischen Heißpunktwert und der Temperatur des Katalysatorfestbetts an der Stelle des Heißpunktwertes wird als Heißpunktausdehnung ΔT^{HB} bezeichnet. Dies ist unter anderem darauf zurückzuführen, dass die Reaktantenkonzentration im Reaktionsgasgemisch am Eingang (Eintritt) des Reaktionsgasgemischs in das Katalysatorfestbett am höchsten ist, was dort besonders hohe Reaktionsgeschwindigkeiten bedingt, mit denen pro Zeiteinheit besonders hohe Reaktionswärmeentwicklung einhergeht (beim Eintritt in das Katalysatorfestbett weist das Reaktionsgasgemisch in der Regel im wesentlichen die Temperatur des Katalysatorfestbetts auf.

Meist benötigen heterogen katalysierte Gasphasen-Partialoxidationen für auf einen einmaligen Durchgang des Reaktionsgasgemischs durch das Katalysatorfestbett bezogene wirtschaftliche Eduktumsätze der Partialoxidation erhöhte Katalysatorfestbetttemperaturen. In der Regel betragen diese einige hundert °C, in typischer Weise 100 bis 600 °C, häufig 150 bis 500 °C, meist 200 bzw. 250 bis 450 °C.

Der Arbeitsdruck kann bei heterogen katalysierten Gasphasen-Partialoxidationen am Katalysatorfestbett unter 1 atm oder über 1 atm liegen. In der Regel liegt er im Bereich von ≥ 1 bis 20, bzw. bis 10 atm. Es ist allgemein bekann, dass heterogen katalysierte Gasphasen-Partialoxidationen wenigstens einer organischen Verbindung an einem in einem Reaktionsraum frisch eingebrachten Katalysatorfestbett im wesentlichen kontinuierlich über längere Zeiträume an ein und demselben Katalysatorfestbett betrieben werden können. Die Reaktionsbedingungen können dabei in der Regel im wesentlichen konstant beibehalten werden.

Allerdings verliert das Katalysatorfestbett dabei im Verlauf der Betriebszeit normalerweise an Qualität. In der Regel verschlechtert sich vor allem die volumenspezifische Aktivität des Katalysatorfestbetts (je höher unter ansonsten unveränderten Reaktionsbedingungen die für einen bestimmten auf den einmaligen Durchgangs des Reaktionsgasgemischs durch das Katalysatorfestbett bezogenen Eduktumsatz erforderliche Temperatur des Katalysatorbetts ist, desto geringer ist die volumenspezifische Aktivität des Katalysatorfestbetts). Meist leidet aber auch die Selektivität der Zielproduktbildung.

Eine abnehmende volumenspezifische Aktivität eines Katalysatorfestbetts ist vor allem deshalb nachteilig, weil sich damit mit zunehmender Betriebszeit des Katalysatorfestbetts unter ansonsten konstanten Betriebsbedingungen der auf den einmaligen Durchgang des Reaktionsgasgemischs durch das Katalysatorfestbett bezogene Eduktumsatz mindert, was die beabsichtigte Raum-Zeit-Ausbeute einer Produktionsanlage an Zielprodukt mindert.

Die EP-A 990 636 und die EP-A 1 106 598 versuchen der vorgenannten Entwicklung im Langzeitbetrieb einer heterogen katalysierten Gasphasen-Partialoxidation wenigstens einer organischen Ausgangsverbindung an ein und demselben Katalysatorfestbett dadurch Rechnung zu tragen, dass die Temperatur des Katalysatorfestbetts im Verlauf der Betriebszeit unter ansonsten weitgehend gleichbleibenden Betriebsbedingungen nach und nach erhöht wird, um den Eduktumsatz bei einmaligem Durchgang des Reaktionsgasgemischs durch das Katalysatorfestbett im wesentlichen beizubehalten.

Als Deaktivierungsrate eines Katalysatorfestbetts wird in dieser Schrift die auf eine Betriebsdauer von einem Jahr (365 Tage) hochgerechnete Erhöhung der Temperatur des Katalysatorfestbetts bezeichnet, die zum Beibehalt des Eduktumsatz bei einmaligem Durchgang des Reaktionsgasgemischs durch das Katalysatorfestbett erforderlich ist (unter ansonsten unveränderten Verfahrensbedingungen).

Nachteilig an der in der EP-A 990 636 sowie in der EP-A 1 106 598 empfohlenen Verfahrensweise ist jedoch, dass sich mit zunehmender Erhöhung der Temperatur des Katalysatorfestbetts sein Alterungsprozess in der Regel beschleunigt, weshalb beim Erreichen eines Höchstwerts der Temperatur des Katalysatorfestbetts das Katalysatorfestbett üblicherweise vollständig ausgetauscht und ein vollständiges unverbrauchtes Katalysatorfestbett frisch in den Reaktionsraum eingebracht wird (die Deaktivierung lässt sich durch eine Erhöhung der Temperatur des Katalysatorfestbetts nicht mehr ausgleichen).

Die DE-A 103 51 269, die DE-A 103 50 812, die DE-A 103 50 822, die EP-A 614 872 und die DE-A 103 50 822 empfehlen, die Notwendigkeit des vollständigen Austauschs des Katalysatorfestbetts dadurch hinauszuzögern, dass das Katalysatorfestbett von Zeit zu Zeit regeneriert wird (d.h., von Zeit zu Zeit das Verfahren der heterogen katalysierten Festbett-Gasphasen-Partialoxidation unterbrechen und z.B. ein heißes Gemisch aus molekularem Sauerstoff und Inertgas durch das Katalysatorfestbett führen). Nachteilig an dieser Verfahrensweise ist jedoch, dass sich ihre Wirksamkeit mit zunehmender Gesamtbetriebsdauer erschöpft.

Die DE-A 10 2004 025 445 empfiehlt als weitere Maßnahme, um die Notwendigkeit des vollständigen Austauschs des Katalysatorfestbetts hinauszuzögern, eine Erhöhung des Arbeitsdrucks in der Gasphase. Nachteilig an dieser Maßnahme ist jedoch, dass sich ihre Wirksamkeit gleichfalls mit zunehmender Gesamtbetriebsdauer erschöpft und dass sie gleichzeitig eine zunehmende Verdichtungsleistung erfordert.

Die DE-A 102 32 748 und die WO 2004/009525 empfehlen als weitere Möglichkeit, um einen vollständigen Austausch des Katalysatorfestbetts hinauszuzögern, nur einen Teil desselben durch ein Ersatzkatalysatorfestbett zu ersetzen, dessen volumenspezifische Aktivität gleich derjenigen des ersetzten Katalysatorfestbettteils in seinem in den Reaktionsraum frisch eingebrachten Zustand sein soll.

Auf diese Art und Weise ist es möglich, den erforderlichen Eduktumsatz (bezogen auf den einmaligen Durchgang des Reaktionsgasgemischs durch das Katalysatorfestbett) unter ansonsten unveränderten Verfahrensbedingungen mit einer vergleichsweise begrenzt erhöhten Temperatur des Katalysatorfestbetts (verglichen mit der für denselben Eduktumsatz mit dem ursprünglichen in den Reaktionsraum frisch eingebrachten Katalysatorfestbett erforderlichen Temperatur des Katalysatorfestbetts) wiederzuerlangen.

Nachteilig an der in der DE-A 102 32 748 und in der WO 2004/009525 beschriebenen Verfahrensweise ist jedoch, dass nach dem Teilersatz des Katalysatorfestbetts die Deaktivierungsrate des nach dem Teilersatz resultierenden Katalysatorfestbetts bei Betrieb desselben bei Temperaturen, die den geforderten Eduktumsatz gewährleisten, erhöht ist (verglichen mit der Deaktivierungsrate des frisch in den Reaktionsraum eingebrachten Katalysatorfestbetts bei ansonsten entsprechender und auf denselben Eduktumsatz zielender Betriebsweise), weshalb die nach dem Teilersatz verfügbare Zeitspanne bis zur Notwendigkeit eines vollständigen Austauschs des Katalysatorfestbetts vergleichsweise beschränkt ist.

Die Aufgabe der vorliegenden Erfindung bestand daher darin, eine verbesserte Ausführungsform eines Teilersatzes eines gebrauchten Katalysatorfestbetts zur Verfügung zu stellen, mit der eine geringere Deaktivierungsrate des nach dem Teilersatz resultierenden Katalysatorfestbetts bei auf gleichen Eduktumsatz zielender und auch im übrigen entsprechender Betriebsweise einhergeht, als dies bei einem Teilersatz gemäß der DE-A 102 32 748 und der WO 2004/009525 der Fall ist.

Demgemäß wurde ein Verfahren der heterogen katalysierten Gasphasen-Partialoxidation wenigstens einer organischen Ausgangsverbindung mit molekularem Sauerstoff an einem in einem Reaktionsraum frisch eingebrachten Katalysatorfestbett, bei dem man zum Zweck der Partialoxidation ein die wenigstens eine organische Ausgangsverbindung und den molekularen Sauerstoff enthaltendes Reaktionsgasgemisch durch das Katalysatorfestbett führt sowie durch indirekten Wärmeaustausch mit einem außerhalb des Reaktionsraums geführten fluiden Wärmeträger Reaktionswämre abführt und dann, wenn mit zunehmender Betriebsdauer eine zunehmende Minderung der Qualität des Katalysatorfestbetts einhergeht, nicht das gesamte Katalysatorfestbett sondern nur ein Teil desselben durch ein Ersatzkatalysatorteilfestbett (mit in der Regel frisch hergestelltem Katalysator) ersetzt, gefunden, das dadurch gekennzeichnet ist, dass die volumenspezifische Aktivität des Ersatzkatalysatorteilfestbetts geringer ist, als die volumenspezifische Aktivität des ersetzten Katalysatorfestbettteils in seinem frisch eingebrachten Zustand.

Als Maß für die volumenspezifische Aktivität einer Katalysatorfestbettschüttung (bzw. eines solchen Abschnitts) wird, wie bereits erwähnt, bei identischem Schüttvolumen die Temperatur der Katalysatorfestbettschüttung herangezogen, die erforderlich ist, um unter ansonsten identischen Verfahrensbedingungen (identische Zusammensetzung des Reaktionsgasgemischs, identische Belastung der Katalysatorfestbettschüttung mit Reaktionsgasgemisch), bezogen auf einen Einmaldurchgang des Reaktionsgasgemischs durch die Katalysatorfestbettschüttung, den (in der großtechnischen Produktion) angestrebten Eduktumsatz zu erzielen. Je höher die erforderliche Temperatur, desto geringer die volumenspezifische Aktivität. Alternativ kann bei identischer Temperatur der Katalysatorfestbettschüttung und identischen sonstigen Verfahrenbedingungen bzw. Betriebsbedingungen (identische Zusammensetzung des Reaktionsgasgemischs, identische Belastung der Katalysatorfestbettschüttung mit Reaktionsgasgemisch) der resultierende, auf Einmaldurchgang durch das Katalysatorfestbett bezogene Eduktumsatz herangezogen werden. Je größer der erzielte Eduktumsatz um so höher ist die volumenspezifische Aktivität.

Die volumenspezifische (d. h., die auf die Einheit des Schüttvolumens normierte) Aktivität kann in einfacher Weise z. B. dadurch verringert werden, dass man eine Grundmenge von einheitlich hergestellten Katalysatorformkörpern mit inerten Verdünnungsformkörpern homogen verdünnt. Je höher der Anteil der inerten Verdünnungsformkörper gewählt wird, desto geringer ist die in einem bestimmten Volumen der Schüttung enthaltene Aktivmasse bzw. Katalysatoraktivität. Unter inerten Verdünnungsformkörpern werden dabei Formkörper aus solchen Materialien verstanden, die sich bezüglich der heterogen katalysierten partiellen Gasphasen-Oxidation im wesentlichen inert verhalten, d. h., möglichst weitgehend keinen Eduktumsatz bedingen. Als solche Materialien kommen für die Mehrzahl der heterogen katalysierten Gasphasen-Partialoxidationen von organischen Ausgangsverbindungen z. B. poröse oder unporöse Aluminiumoxide, Siliciumdioxid, Thoriumdioxid, Zirkondioxid, Siliciumcarbid, Silikate wie Magnesium- oder Aluminiumsilikat oder Steatit in Betracht.

Die Geometrie solcher inerter Verdünnungsformkörper kann im Prinzip beliebig sein. D. h., es können beispielsweise Kugeln, Polygone, Vollzylinder oder Ringe sein. Erfindungsgemäß bevorzugt wird man als inerte Verdünnungsformkörper solche wählen, deren Geometrie derjenigen der mit ihnen zu verdünnenden Katalysatorformkörper entspricht. Eine Abnahme der volumenspezifischen Aktivität ist aber auch z. B. dadurch möglich, dass man bei gleichbleibender Geometrie und Aktivmassenart eines Schalenkatalysatorformkörpers die Dicke der auf dem Träger aufgebrachten Aktivmassenschicht verringert oder in einem Gemisch aus Schalenkatalysatoren mit gleicher Geometrie aber mit unterschiedlichem Gewichtsanteil der Aktivmasse den Anteil an Katalysatorformkörpern mit geringerem Aktivmassengewichtsanteil steigert. Analoge Wirkung lässt sich auch z. B. dadurch erzielen, dass man in Mischungen aus Vollkatalysatoren und aus Schalenkatalysatoren (bei identischer Aktivmasse) in entsprechender Weise das Mischungsverhältnis verändert. Selbstredend lassen sich die beschriebenen Varianten auch kombiniert anwenden. Die volumenspezifische Aktivität kann aber auch dadurch verringert werden, dass man bei gleicher Elementzusammensetzung der Aktivmasse und bei identischen Formgebungsverfahren die spezifische Oberfläche der Aktivmasse z. B. dadurch verringert, dass man die Aktivmasse bei erhöhter Temperatur und/oder über längere Zeit thermisch behandelt.

Selbstverständlich lässt sich die volumenspezifische Aktivität auch dadurch beeinflussen, dass man z. B. bei identischer Formgebung die Elementzusammensetzung der Aktivmasse verändert und z. B. den Anteil derjenigen elementaren Konstituenten verringert, die für eine erhöhte Aktivität besonders förderlich sind. Alternativ kann man auch die Aktivmassen selbst verdünnen, indem man bei der Aktivmassenherstellung z. B. in das thermisch zu behandelnde Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Alle vorgenannten Maßnahmen kommen jeweils für sich oder in beliebiger Kombination zur Steuerung der volumenspezifischen Aktivität des Ersatzkatalysatorteilfestbetts im erfindungsgemäßen Sinn in Betracht. Dazu gehört nicht zuletzt auch die Möglichkeit, bei gleicher geometrischer Form eines Schalenkatalysators sowie gleicher Schalendicke und gleicher Aktivmassenschale die Längstausdehnung des Trägerkörpers (z. B. den Durchmesser der Trägerkugel) zu vergrößern.

Hintergrund der erfindungsgemäßen Lehre ist der Sachverhalt, dass ein in einem Reaktionsraum frisch eingebrachtes Katalysatorfestbett im Verlauf der Betriebszeit einer an ihm durchgeführten heterogen katalysierten Gasphasen-Partialoxidation wenigstens einer organischen Ausgangsverbindung seine Qualität über das Katalysatorfestbett nicht homogen, nicht einheitlich verliert (vgl. z. B. WO 2004/009525). Ursächlich dafür kann z. B. die Heißpunktausbildung und/oder die inhomogene Anreicherung von im Reaktionsgasgemisch enthaltenen Katalysatorgiften (die großtechnischen Reaktionsgasgemische gehen von nicht hochreinen Rohstoffen aus) sein. Unabhängig von der Detailursache erfolgt die Deaktivierung im jeweiligen Katalysatorfestbettabschnitt aber um so rascher, je höher die effektive Temperatur des Katalysatorfestbetts im betreffenden Katalysatorfestbettabschnitt ist.

Ersetzt man nun nach längerer Betriebszeit einen solchermaßen überproportional deaktivierten Abschnitt des Katalysatorfestbetts durch ein Ersatzkatalysatorteilfestbett, dessen volumenspezifische Aktivität der volumenspezifischen Aktivität des ersetzten Kataiysatorfestbettabschnitts in seinem frisch eingebrachten Zustand entspricht, so besteht das Katalysatorgesamtfestbett danach aus zwei Teilabschnitten. Der eine befindet sich im frischen Ursprungszustand, der andere in einem durch vorhergehenden Betrieb unterproportional deaktivierten Zustand. Um mit einem solchen Katalysatorfestbett, bezogen auf den einmaligen Durchgang des Reaktionsgasgemisches, unter ansonsten unveränderten Betriebsbedingungen den selben Eduktumsatz wie bei einem in den Reaktionsraum kompletten frisch eingebrachten Katalysatorfestbett zu erzielen, bedarf es im ersteren Fall einer höheren Katalysatorfestbetttemperatur als im letzteren Fall. Diese bedingt aber im frischen Ersatzkatalysatorteilfestbett eine überproportional erhöhte effektive Katalysatorfestbetttemperatur, da die Aktivität einer frischen Katalysatorbettschüttung mit zunehmender Betriebstemperatur stärker als linear wächst (vgl. EP-A 099 636 und EP-A 1 106 598). Auf den nicht ausgetauschten Katalysatorfestbettabschnitt entfällt ein noch geringerer Umsatzanteil als vorab des Katalysatorteilbettwechsels. Dies bedingt zusammen eine höhere Deaktivierungsrate als im Fall des Betriebs des in den Reaktionsraum in seiner Gesamtheit frisch eingebrachten Katalysatorfestbetts.

Weist das Ersatzkatalysatorteilfestbett hingegen eine geringere volumenspezifische Aktivität als der ersetzte Katalysatorfestbettabschnitt in seinem frisch eingebrachten Zustand auf, bedarf es zur gewünschten Eduktumsatzerlangung zwar einer noch höheren Katalysatorfestbetttemperatur, doch muss in diesem Fall der nicht ausgetauschte Katalysatorfestbettabschnitt einen vergleichsweise erhöhten Umsatzanteil beitragen (selbstredend setzen die vorstehenden Betrachtungen stets einen Beibehalt der Zusammensetzung des Reaktionsgasgemischs sowie der Belastung des Katalysatorfestbetts mit Reaktionsgasgemisch voraus). Damit gehen in der Regel geringere Heißpunktausdehnungen ΔT^{HB}einher und letztlich resultieren im Normalfall geringere Deaktivierungsraten als im Fall eines Ersatzkatalysatorteilfestbetts mit einer volumenspezifischen Aktivität, die derjenigen im ursprünglich frisch eingebrachten Zustand entspricht. Konsequenz einer solchen vergleichsweise verringerten Deaktivierungsrate ist jedoch im Normalfall eine erhöhte Gesamtbetriebsdauer bis ein Komplettaustausch des Katalysatorfestbetts erforderlich ist. D. h., der Charme des erfindungsgemäßen Verfahrens besteht darin, das katalytische Potential des nicht ausgetauschten, zuvor unterforderten Abschnitts des Katalysatorfestbetts möglichst weitgehend zu aktivieren und nachträglich abzurufen. Diese Vorgehensweise schließt ausdrücklich und in erstaunlicher Weise Katalysatorfestbettteilwechsel mit ein, bei denen die volumenspezifische Aktivität des Ersatzkatalysatorteilfestbetts geringer ist, als die volumenspezifische Aktivität des ersetzten Katalysatorfestbettteils zum Zeitpunkt seines Ersatzes. Allerdings sind bzw. umfassen die Katalysatorformkörper im Ersatzkatalysatorteilfestbett normalerweise frisch hergestellten Katalysatorformkörper. Ein weiterer wirtschaftlicher Vorteil der erfindungsgemäßen Verfahrensweise liegt gegenüber der Verfahrensweise des Standes der Technik darin begründet, dass mit einer geringeren volumenspezifischen Aktivität des Ersatzkatalysatorteilfestbetts z. B. ein erhöhter Anteil an inerten Verdünnungsformkörpern einhergeht, was die finanziellen Aufwendungen für das Ersatzkatalysatorteilfestbett verringert. Anstelle den wirtschaftlichen Vorteil einer geringeren Deaktivierungsrate wahrzunehmen, kann selbstredend alternativ auch die Belastung des Katalysatorfestbetts mit Reaktionsgasgemisch erhöht und so z. B. unter Beibehalt der vormaligen Deaktivierungsrate die Raum-Zeit-Ausbeute an Zielprodukt erhöht werden. Erfindungsgemäß vorteilhaft wird man die volumenspezifische Aktivität des Ersatzkatalysatorteilfestbetts so bemessen, dass, bezogen auf gleichen Eduktumsatz bei Einmaldurchgang des Reaktionsgasgemischs durch das Katalysatorfestbett sowie gleiche Reaktionsgasgemischzusammensetzung und Belastung des Katalysatorfestbetts mit Reaktionsgasgemisch, die Differenz dΔT zwischen der Heißpunktausdehnung ΔT^{HB}ₙ nach dem Teilwechsel des Katalysatorfestbetts und der Heißpunktausdehnung ΔT^{HB}ᵥ (unmittelbar) vor dem Teilwechsel des Katalysatorfestbetts (dΔT = ΔT^{HB}ₙ - ΔT^{HB}ᵥ) ≤ 30 °C beträgt. Vorzugsweise wird die volumenspezifische Aktivität des Ersatzkatalysatorteilfestbetts so bemessen, dass dΔT ≤ 25 °C, oder ≤ 20 °C bzw. ≤ 15 °C, besser ≤ 10 oder ≤ 5 °C, besonders vorteilhaft ≤ 0 °C, oder ≤ -5 °C, bzw. ≤ -10 °C, vielfach ≤ -15 °C oder bis zu -20 °C beträgt. In der Regel wird dΔT nicht < - 20 °C betragen. Bevorzugt sind solche erfindungsgemäßen Verfahren, bei denen dΔT-15 bis +10 °C beträgt. Besonders bevorzugt sind solche erfindungsgemäßen Verfahren, bei denen dΔT -10 °C bis 0 °C beträgt. Günstig ist auch ein dΔT von -5 °C bis 0 °C.

Beispielhaft kann der Reaktionsraum des erfindungsgemäßen Verfahren das Innere eines (Kontakt- bzw. Reaktions-)Rohres sein, in welchem das Katalysatorfestbett eingebracht ist und um dessen Außenwand der fluide Wärmeträger geführt wird. Dieser kann grundsätzlich im Gleichstrom, im Gegenstrom, oder im Querstrom zum durch das Reaktionsrohr geführten Reaktionsgasgemisch geführt werden. Zweckmäßig befindet sich das Kontaktrohr in einem Rohrbündelreaktor.

D. h., anwendungstechnisch zweckmäßig wird man die erfindungsgemäße heterogen katalysierte Gasphasen-Partialoxidation der wenigstens einen organischen Ausgangsverbindung großtechnisch in einem Vielkontaktrohr-Festbettreaktor (Rohrbündelreaktor) durchführen. Solche Reaktoren entsprechen in ihrem Typ den Mantel-Rohr-Wärmeaustauschern (grundsätzlich kommt für das erfindungsgemäße Verfahren aber jedwede sonstige Art an bekannten indirekten Wärmeaustauschern zur Aufnahme des Katalysatorfestbetts in Betracht). D. h., ihre übliche Bauart besteht aus einem, in der Regel zylinderförmigen, Behälter, in welchem eine Vielzahl von (normalerweise identischen) (Reaktions)Rohren entsprechend den Kühlrohren eines Mantel-Rohr-Wärmeaustauschers in üblicherweise vertikaler Anordnung untergebracht ist. Diese Kontaktrohre, von denen jedes eine (normalerweise weitestgehend identische) Schüttung des zu verwendenden Katalysatorfestbetts (eine Festbettanordnung der entsprechenden Katalysatorbeschickung) enthält, sind mit ihren Enden üblicherweise in Rohrböden abdichtend befestigt und münden zweckmäßig in je eine am oberen bzw. unteren Ende mit dem Behälter verbundene Haube. Über diese Hauben wird das die Kontaktrohre durchströmende Reaktionsgasgemisch zu- bzw. abgeführt, so dass das Innere eines jeden Kontaktrohres einem langgestreckten erfindungsgemäßen (weitestgehend einheitlichen) Reaktionsraum entspricht.

Durch den die Kontaktrohre umgebenden Raum wird der fluide Wärmeträger (das fluide Wärmeaustauschmittel) geleitet, um die Reaktionswärme (die Prozesswärme) abzuführen (zu bewältigen). Nach dem Austritt aus dem Behälter wird der erwärmte fluide Wärmeträger wieder auf seine ursprüngliche Temperatur gebracht, bevor er wieder dem Reaktionsbehälter zugeführt wird (vgl. z. B. DE-A 30 42 468).

Tritt längs der Kontaktrohre (Reaktionsrohre) auf unterschiedlichen (mehreren) Höhen Wärmeträger (Wärmeaustauschmittel) in den Reaktor ein, so soll in dieser Schrift von einer Anwendung mehrerer Wärmeaustauschmittelkreisläufe oder auch von einem mehrere Temperierzonen aufweisenden Mehrzonenreaktor (Reaktionsraum) gesprochen werden (die einzelnen Kreisläufe sind durch geeignete Trennbleche in der Regel weitgehend voneinander getrennt). Erfolgt der Eintritt des Wärmeträgers (des Wärmeaustauschmittels) lediglich auf einer Höhe (für diese Fälle ist das erfindungsgemäße Verfahren bevorzugt), wird hier von einem Wärmeaustauschmittelkreislauf oder auch von einem Einzonenreaktor gesprochen, selbst wenn dieser Kreislauf nicht mit einer, sondern aus Gründen der Zweckmäßigkeit mit mehreren Pumpen betrieben wird.

D. h., das erfindungsgemäße Verfahren umfasst als eine Ausführungsform insbesondere Verfahren der heterogen katalysierten Gasphasen-Partialoxidation wenigstens einer organischen Ausgangsverbindung mit molekularem Sauerstoff an einem in den Reaktionsräumen (in den Kontaktrohren) eines Vielkontaktrohr-Festbettreaktors frisch eingebrachten Katalysatorfestbett, bei denen man zum Zweck der Partialoxidation ein die wenigstens eine organische Ausgangsverbindung und den molekularen Sauerstoff enthaltendes Reaktionsgasgemisch durch das Katalysatorfestbett führt sowie durch indirekten Wärmeaustausch mit einem außerhalb der Reaktionsräume (Kontaktrohre) geführten fluiden Wärmeträger Reaktionswärme abführt und dann, wenn mit zunehmender Betriebsdauer eine zunehmende Minderung der Qualität des Katalysatorfestbetts einhergeht, zur Rückgewinnung der Qualität des Katalysatorfestbetts im jeweiligen Kontaktrohr nicht das gesamte Katalysatorfestbett sondern nur einen Teil desselben durch ein Ersatzkatalysatorteilfestbett ersetzt, die dadurch gekennzeichnet sind, dass die volumenspezifische Aktivität des Ersatzkatalysatorteilfestbetts geringer ist, als die volumenspezifische Aktivität des ersetzten Katalysatorfestbettteils in seinem frisch eingebrachten Zustand. Dies gilt insbesondere dann, wenn längs der Kontaktrohre lediglich auf einer Höhe Wärmeträger in den Reaktor geführt wird und es sich somit um einen Einzonenreaktor handelt. Insbesondere auf diese beiden vorgenannten Ausführungsformen beziehen sich alle in dieser Schrift über das erfindungsgemäße Verfahren gemachten Aussagen, insbesondere die Quantifizierungen von dΔT.

Beispiele für erfindungsgemäß einsetzbare Einzonen- und Mehrzonen Vielkontaktrohr-Festbett-Reaktoren finden sich z. B. in den Schriften DE-A 100 24 348, DE-A 198 36 792, DE-A 100 32 304, WO 01/87476, DE-A 199 10 508, DE-A 199 10 506, DE-A 199 27 624, DE-A 199 48 241, DE-A 199 48 248, DE-A 199 48 523, DE-A 199 55 168, DE-A 101 34 026, DE-A 101 34 026, DE-A 101 01 695, US-A 5,442,108, EP-A 911 313, EP-A 1 097 745, DE-A 101 37 768, DE-A 101 35 498 und DE-A 100 40 781.

Üblicherweise sind die Kontaktrohre aus ferritischem Stahl gefertigt und weisen häufig eine Wanddicke von 1 bis 3 mm auf. Ihr Innendurchmesser beträgt vielfach 20 bis 30 mm, häufig 21 bis 26 mm. Die Rohrlänge erstreckt sich im Normalfall auf wenige Meter (typisch ist eine Kontaktrohrlänge im Bereich von 2 bis 4 m, häufig 2,5 bis 3,5 m). Davon werden in der Regel wenigstens 60%, häufig wenigstens 75% vom Katalysatorfestbett belegt. Anwendungstechnisch zweckmäßig beläuft sich die im Behälter untergebrachte Anzahl von Kontaktrohren auf wenigstens 5000, vorzugsweise auf wenigstens 10000. Häufig beträgt die Anzahl der im Behälter untergebrachten Kontaktrohre 15000 bis 30000, bzw. bis 40000. Rohrbündelreaktoren mit einer oberhalb von 50000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 30 bis 50 mm, häufig 35 bis 45 mm beträgt (vgl. z. B. EP-A 468 290).

Als fluide Wärmeträger kommen für das erfindungsgemäße Verfahren ganz generell, insbesondere aber im Fall der Anwendung eines Vielkontaktrohr-Festbettreaktors, Salzschmelzen, z. B. die von Salzen wie Kaliumnitrat, Kaliumnitrit, Natriumnitrit und/oder Natriumnitrat in Betracht. Teilweise können aber auch in Abhängigkeit von deren Schmelzpunkt die Schmelzen von niedrig schmelzenden Metallen wie Natrium, Quecksilber sowie von Legierungen verschiedener Metalle verwendet werden.

Das Wärmeaustauschmittel, der Wärmeträger kann dabei in einfacher Weise im wesentlichen direkt längs (im Gleichstrom oder im Gegenstrom zum Reaktionsgasgemisch) zu den Kontaktrohren geführt werden. Es besteht aber auch die Möglichkeit, diese Längsführung (im Gleichstrom oder im Gegenstrom zum Reaktionsgasgemisch) lediglich über den gesamten Behälter betrachtet zu verwirklichen und dieser Längsströmung innerhalb des Behälters durch eine längs der Kontaktrohre aufeinanderfolgende Anordnung von Durchtrittsquerschnitte freilassenden Umlenkscheiben, eine Querströmung so zu überlagern, dass im Längsschnitt durch das Rohrbündel ein mäanderförmiger Strömungsverlauf des Wärmeaustauschmittels resultiert. In der Regel verlässt das Wärmeaustauschmittel den Behälter (Reaktor) mit einer Temperatur, die (bedingt durch die Exothermie der Reaktion) oberhalb seiner Eintrittstemperatur liegt (häufig ≥ 0 bis 10 °C, oft ≥2 bis 8 °C, vielfach ≥ 3 bis 6 °C).

Vorstehende und alle anderen Aussagen zum erfindungsgemäßen Verfahren in dieser Schrift, besitzen insbesondere Gültigkeit für die heterogen katalysierte Festbett-Gasphasen-Partialoxidation von Propylen zu Acrolein und/oder Acrylsäure, von iso-Buten zu Methacrolein und/oder Methacrylsäure, von (Meth)acrolein zu (Meth)acrylsäure, von Propan zu Acrolein und/oder Acrylsäure sowie von iso-Butan zu Methacrolein und/oder Methacrylsäure. Selbstredend gelten sie aber auch für alle anderen eingangs dieser Schrift benannten Partialoxidationen.

Erfindungsgemäß günstig ist es, wenn das beim erfindungsgemäßen Verfahren in den Reaktionsraum frisch eingebrachte Katalysatorfestbett erfindungsgemäß vorteilhaft so gestaltet wird, dass seine volumenspezifische Aktivität in Strömungsrichtung des Reaktionsgasgemischs variiert. Mit besonderem Vorteil wird man es so gestalten, dass seine volumenspezifische Aktivität in Strömungsrichtung des Reaktionsgasgemischs wenigstens einmal abrupt, oder treppenförmig oder kontinuierlich zunimmt.

Mit besonderem Vorteil umfasst das in den Reaktionsraum frisch eingebrachte Katalysatorfestbett in Strömungsrichtung des Reaktionsgasgemischs keine Abnahme der volumenspezifischen Aktivität. Ferner ist es erfindungsgemäß günstig, wenn die Katalysatoren des in den Reaktionsraum frisch eingebrachten Katalysatorfestbetts lediglich eine Aktivmasse aufweisen, die mit besonderem Vorteil zu einer einzigen in diesem Katalysatorfestbett verwendeten Formkörpergeometrie geformt ist. Darüber hinaus ist es erfindungsgemäß günstig, wenn dieser eine vorgenannte Katalysatortyp in seiner frisch hergestellten Form auch als alleiniger Katalysator für das Ersatzkatalysatorteilfestbett verwendet wird.

Es ist weiterhin erfindungsgemäß günstig, wenn innerhalb des in den Reaktionsraum frisch eingebrachten Katalysatorfestbetts lediglich eine Sorte an inertem Verdünnungsformkörper mitverwendet wird. Dieser Verdünnungsformkörper sollte dann in zweckmäßiger Weise auch für das Ersatzkatalysatorteilfestbett verwendet werden. Von besonderer Vorteilhaftigkeit ist die erfindungsgemäße Verfahrensweise somit dann, wenn sich das Ersatzkatalysatorteilfestbett und der durch dieses ersetzte Abschnitt des Katalysatorfestbetts in seinem frisch in den Reaktionsraum eingebrachten Zustand nur durch den erhöhten Anteil an Verdünnungsformkörpern im Ersatzkatalysatorteilfestbett voneinander unterscheiden.

Im folgenden wird die erfindungsgemäße Verfahrensweise ohne irgendeine Beschränkung seiner Allgemeingültigkeit und lediglich beispielhaft am Verfahren einer heterogen katalysierten Festbett-Gasphasen-Partialoxidation von Propylen zu Acrolein und/oder Acrylsäure näher ausgeführt (diese Ausführungen sind jedoch in entsprechender Weise auf andere mögliche erfindungsgemäße Verfahren der heterogen katalysierten Festbett-Gasphasen-Partialoxidation anderer organischer Ausgangsverbindungen und Zielprodukte übertragbar). Alle in dieser Schrift gemachten Aussagen treffen insbesondere auf diese beiden Verfahren zu. Der diesbezüglich erforderliche Rohstoff Propylen wird dabei in der Regel als Bestandteil von "polymer grade" oder "chemical grade" Propylen dem zu verwendenden Reaktionsgasgemisch zugeführt (vgl. WO 2004/009525). Selbstverständlich kann als Proplyenquelle auch eine heterogen katalysierte partielle. Dehydrierung oder Oxidehydrierung von Propan fungieren, wie es z. B. die WO 01/96270 und die DE-A 103 16 039, WO 01/95271, DE-A 33 13 573, WO 03/011804, DE-A 102 45 585 sowie DE-A10 2004 032 129 und DE-A 10 2005 013 039 beschreiben.

Da die heterogen katalysierte Festbett-Gasphasen-Partialoxidation von Propylen zu Acrylsäure in zwei zeitlich aufeinanderfolgenden Schritten über das Acrolein als Zwischenverbindung verläuft, kann sie, wie bereits erwähnt, einstufig oder zweistufig durchgeführt werden.

Abgesehen vom erfindungsgemäßen Katalysatorfestbettteilwechsel kann eine erfindungsgemäße zweistufige heterogen katalysierte Partialoxidation von Propylen zu Acrylsäure unter Verwendung eines Propylen enthaltenden Reaktionsgasausgangsgemischs z. B. wie in den Schriften EP-A 700 714 (erste Reaktionsstufe; wie dort beschrieben, aber auch in entsprechender Gegenstromfahrweise von Salzbad und Reaktionsgasausgangsgemisch über den Rohrbündelreaktor), EP-A 700 893 (zweite Reaktionsstufe; wie dort beschrieben, aber auch in entsprechender Gegenstromfahrweise), WO 04/085369 (insbesondere diese Schrift wird als integraler Bestandteil dieser Schrift betrachtet) (als zweistufiges Verfahren), WO 04/085363, DE-A 103 13 212 (erste Reaktionsstufe), EP-A 1 159 248 (als zweistufiges Verfahren), EP-A 1 159 246 (zweite Reaktionsstufe), EP-A 1 159 247 (als zweistufiges Verfahren), DE-A 199 48 248 (als zweistufiges Verfahren), DE-A 101 01 695 (zweistufig), WO 04/085368 (als zweistufiges Verfahren), DE-A 103 51 269 (zweistufig), DE-A 10 2004 021 764 (zweistufig), WO 04/085362 (erste Reaktionsstufe), WO 04/085370 (zweite Reaktionsstufe), WO 04/085365 (zweite Reaktionsstufe), WO 04/085367 (zweistufig), WO 2004/009525 (zweistufig), EP-A 990 636, EP-A 1 007 007 und EP-A 1 106 598 beschrieben durchgeführt werden.

Dies gilt insbesondere für alle in diesen Schriften enthaltenen Ausführungsbeispiele. Erfolgt beim zweistufigen Verfahren zwischen den beiden Reaktionsstufen eine Zufuhr von molekularem Sekundärsauerstoff, so erfolgt diese vorzugsweise in Form von Luft. Sie kann aber auch als reiner molekularer Sauerstoff oder auch als ein sonstiges Gemisch aus molekularem Sauerstoff und aus Inertgas erfolgen. Vorteilhaft erfolgt die Sekundärsauerstoffzufuhr in solcher Menge, dass das Produktgasgemisch der zweiten Reaktionsstufe (Acrolein → Acrylsäure) noch nicht umgesetzten molekularen Sauerstoff enthält. Allerdings kann die für das Gesamtverfahren benötigte Menge an molekularem Sauerstoff auch bereits dem Reaktionsgasgemisch für die erste Reaktionsstufe (Propylen → Acrolein) zugefügt werden. In der Regel wird das molare Verhältnis von im dem Katalysatorfestbett der ersten Reaktionsstufe zugeführten Reaktionsgasgemisch enthaltenem molekularem Sauerstoff zu in diesem Gemisch enthaltenem Propylen ≥ 1 und ≤ 3 betragen.

Für die jeweilige der beiden Reaktionsstufen geeignete Multimetalloxidkatalysatoren sind vielfach vorbeschrieben und dem Fachmann wohlbekannt. Beispielsweise verweist die EP-A 253 409 auf Seite 5 auf entsprechende US-Patente. Geeignete Katalysatoren für die jeweilige Oxidationsstufe (Reaktionsstufe) offenbaren auch die DE-A 44 31 957, die DE-A 10 2004 025 445 und die DE-A 44 31 949. Dies gilt auch für jene der allgemeinen Formel I in den beiden vorgenannten älteren Schriften. Für die jeweilige Oxidationsstufe (Reaktionsstufe) verwendbare Katalysatoren offenbaren auch die Schriften DE-A 103 25 488, DE-A 103 25 487, DE-A 103 53 954, DE-A 103 44 149, DE-A 103 51 269, DE-A 103 50 812 und DE-A 103 50 822.

Für die erste Reaktionsstufe (Propylen → Acrolein) kommen demnach insbesondere Katalysatoren in Betracht, deren Aktivmasse wenigstens ein Molybdän und/oder Wolfram sowie wenigstens eines der Elemente Wismut, Tellur, Antimon, Zinn und Kupfer enthaltendes Multimetalloxid ist. Unter diesen sind diejenigen bevorzugt, deren Aktivmasse ein Mo, Bi und Fe enthaltendes Multimetalloxid ist.

In der ersten Reaktionsstufe mögliche, Mo, Fe und Bi enthaltende Multimetalloxidaktivmassen sind z. B. die Multimetalloxidaktivmassen der allgemeinen Formel I der DE-A 199 55 176, die Multimetalloxidaktivmassen der allgemeinen Formel I der DE-A 199 48 523, die Multimetalloxidaktivmassen der allgemeinen Formeln I, II und III der DE-A 101 01 695, die Multimetalloxidaktivmassen der allgemeinen Formeln I, II und III der DE-A 199 48 248 und die Multimetalloxidaktivmassen der allgemeinen Formeln I, II und III der DE-A 199 55 168 sowie die in der EP-A 7 00 714 genannten Multimetalloxidaktivmassen. Aber auch alle in der WO 2004/009525 für die erste Reaktionsstufe genannten Mo, Bi und Fe enthaltenden Multimetalloxidmassen kommen in Betracht.

Ferner eignen sich für die erste Reaktionsstufe des erfindungsgemäßen Verfahrens die Mo, Bi und Fe enthaltenden Multimetalloxidkatalysatoren, die in den Schriften Research Disclosure Nr. 497012 vom 29.08.2005, DE-A 100 46 957, DE-A 100 63 162, DE-C 3 338 380, DE-A 199 02-562, EP-A 15 565, DE-C 2 380 765, EP-A 8 074 65, EP-A 27 93 74, DE-A 330 00 44, EP-A 575897, US-A 4438217, DE-A 19855913, WO 98/24746, DE-A 197 46 210 (diejenigen der allgemeinen Formel II),

JP-A 91/294239, EP-A 293 224 und EP-A 700 714 offenbart werden. Dies gilt insbesondere für die beispielhaften Ausführungsformen in diesen Schriften, unter denen jene der EP-A 15565, der EP-A 575897, der DE-A 197 46 210 und der DE-A 198 55 913 besonders bevorzugt werden. Besonders hervorzuheben sind in diesem Zusammenhang ein Katalysator gemäß Beispiel 1 c aus der EP-A 15 565 sowie ein in entsprechender Weise herzustellender Katalysator, dessen Aktivmasse jedoch die Zusammensetzung Mo₁₂Ni_{6,5}Zn₂Fe₂Bi₁P_{0,0065}K_{0,06}Oₓ·10SiO₂ aufweist. Ferner sind hervorzuheben das Beispiel mit der laufenden Nr. 3 aus der DE-A 198 55 913 (Stöchiometrie: Mo₁₂Co₇Fe₃Bi_{0,6}K_{0,08}Si_{1,6}Oₓ) als Hohlzylindervollkatalysator der Geometrie 5 mm x 3 mm x 2 mm (Außendurchmesser x Höhe x Innendurchmesser) sowie der Multimetalloxid II - Vollkatalysator gemäß Beispiel 1 der DE-A 197 46 210. Ferner wären die Multimetalloxid-Katalysatoren der US-A 4,438,217 zu nennen. Letzteres gilt insbesondere dann, wenn diese Hohlzylinder eine Geometrie 5,5 mm x 3 mm x 3,5 mm, oder 5 mm x 2 mm x 2 mm, oder 5mm x 3 mm x 2 mm, oder 6 mm x 3 mm x 3 mm, oder 7 mm x 3 mm x 4 mm (jeweils Außendurchmesser x Höhe x Innendurchmesser) aufweisen. Weitere mögliche Katalysatorgeometrien sind in diesem Zusammenhang Stränge (z. B. 7,7 mm Länge und 7 mm Durchmesser; oder 6,4 mm Länge und 5,7 mm Durchmesser).

Eine Vielzahl von für die ersten Reaktionsstufe geeigneten Mo, Fe und Bi enthaltenden Multimetalloxidaktivmassen lässt sich unter der allgemeinen Formel IV,

Mo₁₂BiₐFe_{b}X¹_{c}X²_{d}X³ₑX⁴_{f}Oₙ (IV),

in der die Variablen nachfolgende Bedeutung aufweisen:
X¹ = Nickel und/oder Kobalt,
X² = Thallium, ein Alkalimetall und/oder ein Erdalkalimetall,
X³ = Zink, Phosphor, Arsen, Bor, Antimon, Zinn, Cer, Blei und/oder Wolfram,
X⁴ = Silicium, Aluminium, Titan und/oder Zirkonium,

a= 0,5 bis 5,
b= 0,01 bis 5, vorzugsweise 2 bis 4,
c= 0 bis 10, vorzugsweise 3 bis 10,
d= 0 bis 2, vorzugsweise 0,02 bis 2,
e= 0 bis 8, vorzugsweise 0 bis 5,
f= 0 bis 10 und
n= eine Zahl, die durch die Wertigkeit und Häufigkeit der von Sauerstoff verschiedenen Elemente in IV bestimmt wird,
subsumieren.

Das Vorgenannte gilt vor allem, wenn sie in an sich bekannter Weise erhalten (siehe z. B. die DE-A 40 23 239) und z. B. in Substanz zu Kugeln, Ringen oder Zylindern geformt oder auch in Gestalt von Schalenkatalysatoren, d. h., mit der Aktivmasse beschichteten vorgeformten, inerten Trägerkörpern, erfindungsgemäß eingesetzt werden. Selbstverständlich gilt das Gesagte aber auch, wenn sie in Pulverform als Katalysatoren für die erste Reaktionsstufe (Propylen → Acrolein) angewendet werden.

Prinzipiell werden Aktivmassen der allgemeinen Formel IV in einfacher Weise in der Regel dadurch hergestellt, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemisch aus Inertgas, NH₃, CO und/oder H₂) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie NH₄OH, (NH₄)₂CO₃, NH₄NO₃, NH₄CHO₂, CH₃COOH, NH₄CH₃CO₂ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die Multimetalloxidaktivmassen der allgemeinen Formel IV können für die erste Reaktionsstufe einer erfindungsgemäßen Partialoxidation von Propylen zu Acrylsäure sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Anstelle von Graphit kann aber auch hexagonales Bornitrid als Hilfsmittel bei der Formgebung verwendet werden, wie es die DE-A 10 2005 037 678 empfiehlt. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders relevante Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung einer erfindungsgemäß geeigneten pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 29 09 671, der EP-A 293 859 oder aus der EP-A 714 700 bekannt ist. Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird häufig im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der Propylenpartialoxidation im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Erfindungsgemäß geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. Erfindungsgemäß relevant ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Erfindungsgemäß zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für den Schritt vom Propylen zum Acrolein erfindungsgemäß geeignete Multimetalloxidaktivmassen sind ferner Massen der allgemeinen Formel V,

[Y¹_{a'}Y²_{b'}·O_{x'}]ₚ[Y³_{c'}·Y⁴_{d'}·Y⁵_{e'}·Y⁶_{f'}Y⁷_{g'}·Y²_{h'}·O_{y'}·]_{q} (V),

in der die Variablen folgende Bedeutung haben:
Y¹ = nur Wismut oder Wismut und wenigstens eines der Elemente Tellur, Antimon, Zinn und Kupfer,
Y² = Molybdän, oder Wolfram, oder Molybdän und Wolfram,
Y³ = ein Alkalimetall, Thallium und/oder Samarium,
Y⁴ = ein Erdalkalimetall, Nickel, Kobalt, Kupfer, Mangan, Zink, Zinn, Cadmium und/oder Quecksilber,
Y⁵ = Eisen oder Eisen und wenigstens eines der Elemente Chrom und Cer,
Y⁶= Phosphor, Arsen, Bor und/oder Antimon,
Y⁷ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

a' = 0,01 bis 8,
b' = 0,1 bis 30,
c' = 0 bis 4,
d' = 0 bis 20,
e' > 0 bis 20,
f' = 0 bis 6,
g' = 0 bis 15,
h' =8 bis 16,
x',y'= Zahlen, die durch die Wertigkeit und Häufigkeit der von Sauerstoff verschiedenen Elemente in V bestimmt werden und
p,q = Zahlen, deren Verhältnis p/q 0,1 bis 10 beträgt,
enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Y¹_{a'}Y²_{b'}O_{x'}, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders geeignete Multimetalloxidmassen V sind erfindungsgemäß solche, in denen Y¹ nur Wismut ist.

Unter diesen sind wiederum jene von besonderer Relevanz, die der allgemeinen Formel VI,

[Bi_{a"}Z²_{b"}O_{x"}]_{p"} [Z²₁₂Z³_{c"}Z⁴_{d"}Fe_{e"}Z⁵_{f"}Z⁶_{g"}Z⁷_{h"}O_{y"}]_{q"} (VI),

in der die Variablen folgende Bedeutung haben:
Z² = Molybdän, oder Wolfram, oder Molybdän und Wolfram,
Z³ = Nickel und/oder Kobalt,
Z⁴ = Thallium, ein Alkalimetall und/oder ein Erdalkalimetall,
Z⁵= Phosphor, Arsen, Bor, Antimon, Zinn, Cer und/oder Blei,
Z⁶ = Silicium, Aluminium, Titan und/oder Zirkonium,
Z⁷= Kupfer, Silber und/oder Gold,

a" = 0,1 bis 1,
b" = 0,2 bis 2,
c" = 3 bis 10,
d" = 0,02 bis 2,
e" = 0,01 bis 5, vorzugsweise 0,1 bis 3,
f" = 0 bis 5,
g" = 0 bis 10,
h" = 0 bis 1,
x",y"= Zahlen, die durch die Wertigkeit und Häufigkeit der von Sauerstoff verschiedenen Element in VI bestimmt werden,
p",q"=Zahlen, deren Verhältnis p"/q" 0,1 bis 5, vorzugsweise 0,5 bis 2 beträgt,
entsprechen, wobei diejenigen Massen VI ganz besonders bevorzugt werden, in denen Z²_{b}" = (Wolfram)_{b"} und Z²₁₂ = (Molybdän)12 ist.

Ferner ist es erfindungsgemäß von Bedeutung, wenn wenigstens 25 mol-% (bevorzugt wenigstens 50 mol-% und besonders bevorzugt 100 mol-%) des gesamten Anteils [Y¹_{a'}Y²_{b'}O_{x'}]ₚ ([Bi_{a"}Z²_{b"}O_{x"}]_{p"}) der erfindungsgemäß geeigneten Multimetalloxidmassen V (Multimetalloxidmassen VI) in den erfindungsgemäß geeigneten Multimetalloxidmassen V (Multimetalloxidmassen VI) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung Y¹_{a'}Y²_{b'}O_{x'}[Bi_{a"}Z²_{b"}O_{x"}] vorliegen, deren Größtdurchmesser im Bereich 1 nm bis 100 µm liegt.

Hinsichtlich der Formgebung gilt bezüglich Multimetalloxidmassen V-Katalysatoren das bei den Multimetalloxidmassen IV-Katalysatoren Gesagte.

Die Herstellung von Multimetalloxidmassen V-Aktivmassen ist z. B. in der EP-A 575 897 sowie in der DE-A 198 55 913 beschrieben.

Die vorstehend empfohlenen inerten Trägermaterialien kommen u.a. auch als inerte Materialien zur Verdünnung und/oder Abgrenzung der entsprechenden Katalysatorfestbetten, bzw. als deren schützende und/oder das zugeführte Reaktionsgasgemisch aufheizende Vorschüttung in Betracht.

Für den zweiten Schritt (die zweite Reaktionsstufe), die heterogen katalysierte Gasphasen-Partialoxidation von Acrolein zu Acrylsäure, kommen erfindungsgemäß prinzipiell alle Mo und V enthaltenden Multimetalloxidmassen als Aktivmassen für die benötigten Katalysatoren in Betracht, z. B. jene der DE-A 100 46 928 und der DE-A 198 15 281.

Eine Vielzahl derselben, die erfindungsgemäß besonders günstig sind, lässt sich unter der allgemeinen Formel VII,

Mo₁₂VₐX¹_{b}X²_{c}X³_{d}X⁴ₑX⁵_{f}X⁶_{g}Oₙ (VII),

in der die Variablen folgende Bedeutung haben:
X¹ = W, Nb, Ta, Cr und/oder Ce,
X² = Cu, Ni, Co, Fe, Mn und/oder Zn,
X³ = Sb und/oder Bi,
X⁴ = eines oder mehrere Alkalimetalle,
X⁵ = eines oder mehrere Erdalkalimetalle,
X⁶ = Si, Al, Ti und/oder Zr,

a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in VII bestimmt wird,
subsumieren.

Erfindungsgemäß besonders günstige Ausführungsformen innerhalb der aktiven Multimetalloxide VII sind jene, die von den nachfolgenden Bedeutungen der Variablen der allgemeinen Formel VII erfasst werden:
X¹ = W, Nb, und/oder Cr,
X² = Cu, Ni, Co, und/oder Fe,
X³ = Sb,
X⁴ = Na und/oder K,
X⁵= Ca, Sr und/oder Ba,
X⁶ = Si, Al, und/oder Ti,

a = 1,5 bis 5,
b = 0,5 bis 2,
c = 0,5 bis 3,
d = 0 bis 2,
e = 0 bis 0,2,
f = 0 bis 1 und
n = eine Zahl, die durch die Wertigkeit und Häufigkeit der von Sauerstoff verschiedenen Elemente in VII bestimmt wird.

Erfindungsgemäß ganz besonders günstige Multimetalloxide VII sind jedoch jene der allgemeinen Formel VIII,

Mo₁₂V_{a'}Y¹_{b'}Y²_{c'}Y⁵_{f'}Y⁶_{g'}O_{n'} (VIII),

mit
Y¹ = W und/oder Nb,
Y² = Cu und/oder Ni,
Y⁵ = Ca und/oder Sr,
Y⁶ = Si und/oder Al,

a' = 2 bis 4,
b' = 1 bis 1,5,
c'= 1 bis 3,
f' = 0 bis 0,5
g' = 0 bis 8 und
n' = eine Zahl, die durch die Wertigkeit und Häufigkeit der von Sauerstoff verschiedenen Elementen in VIII bestimmt wird.

Die erfindungsgemäß geeigneten Multimetalloxidaktivmassen (VII) sind in an sich bekannter, z. B. in der DE-A 43 35 973 oder in der EP-A 714 700 offenbarter, Weise erhältlich.

Generell können für den Schritt "Acrolein → Acrylsäure" erfindungsgemäß geeignete Multimetalloxidaktivmassen, insbesondere solche der allgemeinen Formel VII, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemische aus Inertgas und reduzierenden Gasen wie H₂, NH₃, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen VII kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen VII kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die resultierenden Multimetalloxidmassen, insbesondere jene der allgemeinen Formel VII, können für eine erfindungsgemäße Acroleinpartialoxidation sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Günstige Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm (z. B. 8,2 mm oder 5,1 mm) betragen kann.

Natürlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 2 909 671, der EP-A 293 859 oder aus der EP-A 714 700 bekannt ist.

Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird in erfindungsgemäß relevanter Weise häufig im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z. B. Kugeln oder Hohlzylinder mit Splittauflage, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. D. h., geeignete Kugelgeometrien können Durchmesser von 8,2 mm oder von 5,1 mm aufweisen. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Relevant sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für den Partialoxidationsschritt "Acrolein → Acrylsäure" geeignete Multimetalloxidaktivmassen sind ferner Massen der allgemeinen Formel IX,

[D]ₚ[E]_{q} (IX),

in der die Variablen folgende Bedeutung haben:
D = Mo₁₂V_{a"}Z¹_{b"}Z²_{c"}Z³_{d"}Z⁴_{e"}Z⁵_{f"}Z⁶_{g"}O_{x"},
E = Z⁷₁₂Cu_{h"}H_{i"}O_{y"} ,
Z¹ = W, Nb, Ta, Cr und/oder Ce,
Z² = Cu, Ni, Co, Fe, Mn und/oder Zn,
Z³ = Sb und/oder Bi,
Z⁴ = Li, Na, K, Rb, Cs und/oder H
Z⁵ = Mg, Ca, Sr und/oder Ba,
Z⁶ = Si, Al, Ti und/oder Zr,
Z⁷ = Mo, W, V, Nb und/oder Ta, vorzugsweise Mo und/oder W

a" = 1 bis 8,
b" = 0,2 bis 5,
c" = 0 bis 23,
d" = 0 bis 50,
e" = 0 bis 2,
f" = 0 bis 5,
g" = 0 bis 50,
h" = 4 bis 30,
i" = 0 bis 20 und
x",y" = Zahlen, die durch die Wertigkeit und Häufigkeit der von Sauerstoff verschiedenen Element in IX bestimmt werden und
p,q = von Null verschiedene Zahlen, deren Verhältnis p/q 160:1 bis 1:1 beträgt,
und die dadurch erhältlich sind, dass man eine Multimetalloxidmasse E

Z⁷₁₂Cu_{h"}H_{i"}O_{y"} (E),

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, Z¹, Z², Z³, Z⁴, Z⁵, Z⁶, die die vorgenannten Elemente in der Stöchiometrie D

Mo₁₂V_{a"}Z¹_{b"}Z²_{c"}Z³_{d"}Z⁴_{e"}Z⁵_{f"}Z⁶_{g"} (D),

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Besonders geeignet sind die Multimetalloxidmassen IX, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen VI-Katalysatoren enthalten z. B. die EP-A 668 104, die DE-A 197 36 105, die DE-A 100 46 928, die DE-A 197 40 493 und die DE-A 195 28 646.

Hinsichtlich der Formgebung gilt das bezüglich Multimetalloxidmassen IX-Katalysatoren Gesagte. Für den Schritt "Acrolein → Acrylsäure" erfindungsgemäß besonders geeignete Multimetalloxidkatalysatoren sind auch jene der DE-A 198 15 281, insbesondere mit Multimetalloxidaktivmassen der allgemeinen Formel I der DE-A 198 15 281.

Mit Vorteil werden für den Schritt vom Propylen zum Acrolein Vollkatalysatorringe und für den Schritt vom Acrolein zur Acrylsäure Schalenkatalysatorringe verwendet.

Die Katalysatorfestbetttemperatur für die erste Reaktionsstufe (Propylen → Acrolein) beträgt zweckmäßig 270 bis 450 °C, bzw. 280 bis 420 °C, vorzugsweise 300 bis 380 °C. Die Katalysatorfestbetttemperatur für die zweite Reaktionsstufe (Acrolein → Acrylsäure) beträgt zweckmäßig 200 bis 370 °C, bzw. 200 bis 320 °C, vorzugsweise 220 bis 380 °C. Bei Durchführung des erfindungsgemäßen Verfahrens in Einzonen Vielkontaktrohr-Festbettreaktoren entsprechen vorgenannte Temperaturen der Eintrittstemperatur des Wärmeträgers (der Salzschmelze) in den die Kontaktrohre umgebenden Behälter.

Prinzipiell kann beim erfindungsgemäßen Verfahren innerhalb des in den Reaktionsraum frisch eingebrachten Katalysatorfestbetts für die erste Reaktionsstufe die volumenspezifische Aktivität in Strömungsrichtung des Reaktionsgasgemischs über die Länge des Strömungsweges (d.h., über die Länge des Katalysatorfestbetts) konstant sein, oder vorteilhaft wenigstens einmal (kontinuierlich, oder abrupt oder stufenförmig) zunehmen. In allen vorgenannten Fällen ist es ferner von Vorteil, wenn sich die Aktivmassenzusammensetzung über die Länge des Strömungsweges (d.h., innerhalb des Katalysatorfestbetts) nicht ändert. Das für die erste Reaktionsstufe vorgenannte gilt ebenso für die zweite Reaktionsstufe einer heterogen katalysierten Festbett-Gasphasen-Partialoxidation von Propylen zu Acrylsäure.

Zur Bereitung des in den Reaktionsraum frisch eingebrachten Katalysatorfestbetts für die erste Reaktionsstufe können nur Multimetalloxidaktivmasse aufweisende Katalysatorformkörper oder auch weitgehend homogene Gemische aus Multimetalloxidaktivmasse aufweisenden Katalysatorformkörpern und keine Multimetalloxidaktivmasse aufweisenden, sich bezüglich der heterogen katalysierten partiellen Gasphasenoxidation im wesentlichen inert verhaltenden, Formkörpern (Verdünnungsformkörper) verwendet werden. Als Materialien für solche inerten Formkörper kommen prinzipiell alle diejenigen in Betracht, die sich auch als Trägermaterial für erfindungsgemäß geeignete Schalenkatalysatoren eignen. Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit in Betracht.

Die Geometrie solcher inerter Verdünnungsformkörper kann im Prinzip beliebig sein. D.h., es können beispielsweise Kugeln, Polygone, Vollzylinder oder auch, wie die Aktivmasse aufweisenden Katalysatorformkörper, Ringe sein. Erfindungsgemäß bevorzugt wird man als inerte Verdünnungsformkörper solche wählen, deren Geometrie derjenigen der mit ihnen zu verdünnenden Katalysatorformkörper im wesentlichen entspricht (vorgenannte Aussagen gelten so auch für zur Bereitung des Katalysatorfestbetts für die zweite Reaktionsstufe verwendbare weitgehend homogene Gemische aus Multimetalloxidaktivmasse aufweisenden Katalysatorformkörpern und Verdünnungsformkörpern).

Vorteilhaft ist es, wenn sich die chemische Zusammensetzung der verwendeten Aktivmasse über das in den Reaktionsraum frisch eingebrachte Katalysatorfestbett für die erste Reaktionsstufe (die Festbettkatalysatorschüttung 1) nicht verändert. D.h., die für einen einzelnen Katalysatorformkörper verwendete Aktivmasse kann zwar ein Gemisch aus verschiedenen Multimetalloxiden sein, für alle Katalysatorformkörper der Festbettkatalysatorschüttung 1 ist dann jedoch bevorzugt das gleiche Gemisch zu verwenden.

Die volumenspezifische (d.h., die auf die Einheit des Volumens normierte) Aktivität kann, wie bereits gesagt, in einfacher Weise dadurch verringert werden, dass man eine Grundmenge von einheitlich hergestellten Katalysatorformkörpern mit Verdünnungsformkörpern homogen verdünnt. Je höher der Anteil der Verdünnungsformkörper gewählt wird, desto geringer ist die in einem bestimmten Volumen der Schüttung enthaltene Aktivmasse bzw. Katalysatoraktivität.

Eine in Strömungsrichtung des Reaktionsgasgemisches über die Festbettkatalysatorschüttung 1 wenigstens einmal zunehmende volumenspezifische Aktivität lässt sich für das erfindungsgemäße Verfahren somit in einfacher Weise z.B. dadurch einstellen, dass man die Schüttung mit einem hohen Anteil an inerten Verdünnungsformkörpern bezogen auf eine Sorte von Katalysatorformkörpern beginnt, und dann diesen Anteil an Verdünnungsformkörpern in Strömungsrichtung entweder kontinuierlich oder wenigstens einmal oder mehrfach abrupt (z.B. stufenförmig) verringert. Lässt man den Anteil an Verdünnungsformkörpern konstant oder werden überhaupt keine Verdünnung formkörper in der Festbettkatalysatorschüttung 1 mitverwendet, resultiert eine konstante volumenspezifische Aktivität in Strömungsrichtung des Reaktionsgasgemisches über die Festbettkatalysatorschüttung 1. Eine Zunahme der volumenspezifischen Aktivität ist aber auch z.B. dadurch möglich, dass man bei gleichbleibender Geometrie und Aktivmassenart eines Schalenkatalysatorformkörpers die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse den Anteil an Katalysatorformkörpern mit höherem Aktivmassengewichtsanteil steigert. Analoge Wirkung lässt sich auch z.B. dadurch erzielen, dass man in Mischungen aus Vollkatalysatoren und aus Schalenkatalysatoren (bei identischer Aktivmasse) in entsprechender Weise das Mischungsverhältnis verändert. Selbstredend lassen sich die beschriebenen Varianten auch kombiniert anwenden.

Im Normalfall wird bei einer erfindungsgemäßen zweistufigen Partialoxidation von Propylen zu Acrylsäure weder innerhalb der Festbettkatalysatorschüttung 1 noch innerhalb der Festbettkatalysatorschüttung 2 (das ist das in den Reaktionsraum frisch eingebrachte Katalysatorfestbett für die zweite Reaktionsstufe) in Strömungsrichtung des Reaktionsgasgemisches die volumenspezifische Aktivität einmal abnehmen.

Vorab und/oder im Anschluss an die Festbettkatalysatorschüttung 1 können sich ausschließlich aus Inertmaterial (z.B. nur Verdünnungsformkörpern) bestehende Schüttungen befinden (sie werden in dieser Schrift begrifflich nicht der Festbettkatalysatorschüttung 1 zugerechnet, da sie keine Formkörper enthalten, die Multimetalloxidaktivmasse aufweisen). Dabei können die für die Inertschüttung verwendeten Verdünnungsformkörper die gleiche Geometrie wie die in der Festbettkatalysatorschüttung 1 verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Erfindungsgemäß bevorzugt ist beim erfindungsgemäßen Verfahren die Festbettkatalysatorschüttung 1 in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h. z.B. auf einer Länge von 0,70 bis 1,50 m, bevorzugt 0,90 bis 1,20 m), jeweils der Gesamtlänge der Festbettkatalysatorschüttung 1 entweder nur Katalysatorformkörper, oder ein homogenes Gemisch (oder zwei solche mit abnehmender Verdünnung aufeinander folgende homogene Gemische) aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diese erste Zone der Festbettkatalysatorschüttung 1 befindet sich dann erfindungsgemäß vorteilhaft bis zum Ende der Länge der Festbettkatalysatorschüttung 1 (d.h., z.B. auf einer Länge von 2,00 bis 3,50 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige (unverdünnte) Schüttung derselben Katalysatorformkörper, die auch in der ersten Zone verwendet worden sind.

Das Vorgenannte trifft insbesondere dann zu, wenn in der Festbettkatalysatorschüttung 1 als Katalysatorformkörper Vollkatalysatorringe oder Schalenkatalysatorringe (insbesondere jene, die in dieser Schrift als bevorzugt genannt werden) eingesetzt werden. Mit Vorteil weisen im Rahmen der vorgenannten Strukturierung sowohl die Katalysatorformkörper als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

In entsprechender Weise wie die volumenspezifische Aktivität der Festbettkatalysatorschüttung 1 variiert werden kann, kann auch die volumenspezifische Aktivität der Festbettkatalysatorschüttung 2 variiert werden. Dabei kann sich vorab und/oder im Anschluss an die eigentliche Festbettkatalysatorschüttung 2 wiederum eine entsprechende Inertschüttung (sie wird in dieser Schrift begrifflich nicht der Festbettkatalysatorschüttung 2 zugerechnet, da sie keine Formkörper enthalten, die Multimetalloxidaktivmasse aufweisen) befinden.

Erfindungsgemäß bevorzugt ist beim erfindungsgemäßen Verfahren die Festbettkatalysatorschüttung 2 in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge der Festbettkatalysatorschüttung 2, entweder nur Katalysatorformkörper oder ein homogenes Gemisch (oder zwei solche mit abnehmender Verdünnung aufeinander folgende homogene Gemische) aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 10 bis 50 Gew.-%, vorzugsweise 20 bis 45 Gew.-% und besonders bevorzugt 25 bis 35 Gew.-% beträgt. Im Anschluss an diese erste Zone der Festbettkatalysatorschüttung 2 befindet sich dann erfindungsgemäß vorteilhaft bis zum Ende der Länge der Festbettkatalysatorschüttung 2 (d.h., z.B. auf einer Länge von 2,00 bis 3,50 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige Schüttung derselben Katalysatorformkörper, die auch in der ersten Zone verwendet worden sind.

Das Vorgenannte trifft insbesondere dann zu, wenn in der Festbettkatalysatorschüttung 2 als Katalysatorformkörper Schalenkatalysatorringe eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 7 mm x 3 mm x 4 mm (Außendurchmesser x Länge x Innendurchmesser) auf.

Generell wird man das in den Reaktionsraum frisch eingebrachte Katalysatorfestbett und die übrigen Randbedingungen für beide Reaktionsstufen so gestalten, dass, wie in der EP-A 990 636 und in der EP-A 1 106 598 beschrieben, sowohl die Heißpunktausbildung als auch deren Temperatursensitivität möglichst gering sind (ΔT^{HB} ist in der Regel ≤ 80 °C, meist ≤70 °C, häufig 20 bis 70 °C, vorzugsweise ist ΔH^{HB} gering; die "peak-to-salt temperature sensitivity" beträgt meist ≤ 9 °C, oder ≤ 7 °C, oder ≤ 5 °C, oder ≤ 3 °C).

Selbstverständlich können vorab eines erfindungsgemäßen Katalysatorfestbettteilwechsel alle im Stand der Technik genannten Verfahrensweisen jeweils für sich oder in Kombination angewendet werden, die geeignet sind, um das Erfordernis eines erfindungsgemäßen Teilwechsels zeitlich hinauszuzögern.

Anwendungstechnisch zweckmäßig wird man sowohl die erste als auch die zweite Reaktionsstufe mit den vorstehend dafür als geeignet beschriebenen, jeweils in den Reaktionsraum frisch einzubringenden Katalysatorfestbetten in einem in dieser Schrift dafür bereits als geeignet beschriebenen Einzonen-Vielkontaktrohr-Festbettreaktor durchführe (betreiben). Diesbezüglich bevorzugt geeignete Einzonen-Vielkontaktrohr-Festbettreaktoren beschreiben die EP-A 700 714 und die EP-A 700 893. Grundsätzlich kann für beide Reaktionsstufen aber auch eine Mehrzonen-Vielkontaktrohr-Festbettreaktor Betriebsweise angewendet werden, wie sie z.B. in der DE-A 103 13 213, der DE-A10 2005 062 026, der WO 2004/009525 und in der DE-A 103 51 269 beschrieben ist.

Sowohl in der ersten als auch in der zweiten Reaktionsstufe kann der Arbeitsdruck sowohl unterhalb von Normaldruck (z.B. bis zu 0,5 bar; das Reaktionsgasgemisch wird durch das Katalysatorfestbett durchgesaugt) als auch oberhalb von Normaldruck liegen. Typischerweise wird der Arbeitsdruck in beiden Reaktionsstufen bei Werten von 1 bis 5 bar, häufig 1,5 bis 3,5 bar liegen. Normalerweise wird der Arbeitsdruck in beiden Reaktionsstufen 100 bar nicht überschreiten.

Die Propylenbelastung der Festbettkatalysatorschüttung 1 kann ≥ 80 Nl/l·h, oder ≥ 100 Nl/l·h, oder ≥ 120 Nl/l·h, oder ≥ 140 Nl/l·h, oder ≥ 165 Nl/l·h, oder ≥ 170 Nl/l·h, oder ≥ 175 Nl/l·h, oder ≥ 180 Nl/l·h, oder ≥ 185 Nl/l·h, oder ≥ 190 Nl/l·h, bzw. ≥ 200 Nl/l·h, oder ≥ 210 Nl/l·h, oder ≥ 220 Nl/l·h, oder ≥ 230 Nl/l·h, oder ≥ 240 Nl/l·h, oder ≥ 250 Nl/l·h betragen. Normalerweise wird die Propylenbelastung der Festbettkatalysatorschüttung 1 600 Nl/l·h nicht überschreiten. In typischer Weise liegen die Propylenbelastungen der Festbettkatalysatorschüttung 1 bei Werten ≤ 300 Nl/l·h, häufig bei Werten ≤ 250 Nl/l·h.

Die Gesamtraumbelastung kann in beiden Reaktionsstufen dabei z. B. 1000 bis 3000 Nl/l·h betragen. Für die Acroleinbelastung der Festbettkatalysatorschüttung 2 gilt das Vorgenannte in gleicher Weise.

Als Quelle für den in beiden Reaktionsstufen benötigten molekularen Sauerstoff kommt sowohl Luft, als auch an molekularem Stickstoff entreicherte Luft oder reiner molekularer Sauerstoff in Betracht. Im Reaktionsgasgemisch für die erste Reaktionsstufe wird das molare Verhältnis O₂ : Propylen in der Regel ≥ 1 betragen. Im Reaktionsgasgemisch für die zweite Stufe wird das molare Verhältnis O₂ : Acrolein in der Regel ≥ 0,5 betragen. In beiden Stufen ist das molare Verhältnis üblicherweise ≤ 3.

Das Reaktionsgasgemisch, mit dem die Festbettkatalysatorschüttung 1 beschickt wird (in dieser Schrift auch Reaktionsgasgemisch 1 genannt), wird in der Regel folgende Bestandteilsvolumen (in Nl/l·h) verhältnisse aufweisen: Propylen : Sauerstoff : Inertgase (einschließlich Wasserdampf) = 1 : (1,0 bis 3,0) : (5 bis 25), vorzugsweise 1 : (1,7 bis 2,3) : (10 bis 15).

Das Reaktionsgasgemisch, mit dem die Festbettkatalysatorschüttung 2 beschickt wird (in dieser Schrift auch Reaktionsgasgemisch 2 genannt), wird in der Regel folgende Bestandteilsvolumen (in Nl/l·h) verhältnisse aufweisen: Acrolein : Sauerstoff : Wasserdampf : Inertgase (ausschließlich Wasserdampf) = 1 : (0,5 bis 3) : (0 bis 20) : (3 bis 30), vorzugsweise 1 : (1 bis 3) : (0,5 bis 10) : (7 bis 18).

Prinzipiell können beide Reaktionsstufen unabhängig voneinander betrieben werden. Häufig wird jedoch das Produktgasgemisch der ersten Stufe zur Beschickung der zweiten Reaktionsstufe verwendet. Dabei hat es sich als zweckmäßig erwiesen, das die erste Reaktionsstufe verlassende Produktgasgemisch vor dem Eintritt in die zweite Reaktionsstufe abzukühlen, um sie eine Nachverbrennung von Teilen des in der ersten Reaktionsstufe gebildeten Acroleins zu unterdrücken. Üblicherweise wird dazu zwischen beide Reaktionsstufen ein Nachkühler geschaltet. Dies kann im einfachsten Fall ein indirekter Rohrbündelwärmeüberträger sein. Zwischen den beiden Reaktionsstufen kann Sekundärgas (molekularer Sauerstoff und/oder Inertgas) zudosiert werden. Häufig wird dem Produktgasgemisch der ersten Reaktionsstufe Luft zudosiert, bevor es zur Beschickung der zweiten Reaktionsstufe verwendet wird. Anwendungstechnisch zweckmäßig wird das Reaktionsgasgemisch der Festbettkatalysatorschüttung 1 auf die Temperatur des Katalysatorfestbetts für die erste Reaktionsstufe vorerwärmt zugeführt.

Im vorgenannten Nachkühler wird das Produktgasgemisch der ersten Reaktionsstufe in der Regel auf eine Temperatur von 210 bis 290 °C, häufig 230 bis 280 °C, oder 250 bis 270 °C abgekühlt. Dabei kann die Abkühlung durchaus auf Temperaturen erfolgen, die unterhalb der Temperatur des Katalysatorfestbetts der zweiten Reaktionsstufe liegen. Günstig ist es, wenn sowohl das Produktgasgemisch der ersten Reaktionsstufe als auch das der zweiten Reaktionsstufe noch bis zu 5 Vol.-%, häufig bis zu 3 Vol.-% an überschüssigem molekularem Sauerstoff enthält.

Die beschriebene Nachkühlung ist jedoch keineswegs zwingend und kann insbesondere dann in aller Regel entfallen, wenn der Weg des Produktgasgemischs von der ersten Reaktionsstufe in die zweite Reaktionsstufe kurz gehalten wird. Ebenso ist eine Zugabe von Sekundärgas zwischen beiden Reaktionsstufen nicht obligatorisch. Sie wird normalerweise insbesondere dann entfallen, wenn man zwei Einzonen-Vielkontaktrohr-Festbettreaktoren für die beiden Reaktionsstufen zu einem dann Zweizonen-Vielkontaktrohr-Festbettreaktor (auch "Single Reactor" genannt) verschmilzt, wie er z.B. in der DE-C 28 30 765, in der EP-A 911 313 und in der EP-A 383 224 beschrieben ist. In diesem Fall wird die erste Reaktionsstufe in der ersten Temperaturzone und die zweite Reaktionsstufe in der zweiten Temperaturzone eines Zweizonen-Vielkontaktrohr-Festbettreaktors verwirklicht und das Reaktionsgasgemisch für die erste Reaktionsstufe enthält den gesamten molekularen Sauersfoffbedarf zugesetzt. Die Wärmeträger der beiden Zonen sind dabei in der Regel durch ein entsprechendes Trennblech im wesentlichen voneinander getrennt. Die Länge der Reaktionsrohre entspricht in den Verschmelzungsfällen vielfach der Summe der Längen in entsprechenden nicht verschmolzenen Rohrbündelreaktoren.

In der Regel wird die erste Reaktionsstufe so betrieben, dass der Propylenumsatz U^{P} bei einmaligem Durchgang des Reaktionsgasgemischs ≥ 90 mol-% und die Selektivität der Acrolein- sowie der Acrylsäurenebenprodtiktbildung (bezogen auf umgesetztes Propylen) zusammengenommen (S^{AC}) ≥ 80 mol-% beträgt. Bevorzugt beträgt U^{P} ≥ 93 mol-%, S^{AC} beträgt mit Vorteil ≥ 85 mol-%, bzw. ≥ 90 mol-%, oder ≥ 95 mol-%.

In entsprechender Weise wird die zweite Reaktionsstufe in der Regel so betrieben, dass der Acroleinumsatz U^{A} bei einmaligem Durchgang des Reaktionsgasgemischs ≥ 90 mol-%, oft ≥93 mol-%, vielfach ≥ 95 mol-%, oder ≥ 97 mol-%, bzw. 99 mol-% beträgt.

Die Selektivität der Acrylsäurebildung (bezogen auf umgesetztes Acrolein) wird dabei regelmäßig ≥ 90 mol-%, oft ≥ 93 mol-% und meist ≥ 96 mol-% betragen.

Günstig ist es, die erste Reaktionsstufe so zu betreiben, dass der Propylengehalt im Produktgasgemisch dieser Stufe den Wert 10000 Gew.-ppm, vorzugsweise 6000 Gew.-ppm und besonders bevorzugt 4000 bzw. 2000 Gew.-ppm nicht übersteigt.

Günstig ist es, die zweite Reaktionsstufe so zu betreiben, dass der Acroleingehalt im Produktgasgemisch dieser Stufe den Wert 1500 Gew.-ppm, vorzugsweise 600 Gew.-ppm und besonders bevorzugt 350 Gew.-ppm nicht übersteigt.

In der Regel enthält das Reaktionsgasgemisch für die erste Reaktionsstufe (hier auch Reaktionsgasausgangsgemisch 1 genannt) beim erfindungsgemäßen Verfahren 3 bis 25 Vol.-%, vielfach 5 bis 20 Vol.-%, und meist 6 bis 13 Vol.-%, Propylen.

Entsprechende Acroleingehalte enthält in der Regel das Reaktionsgasgemisch 2 zur Beschickung der zweiten Reaktionsstufe.

Der Gehalt an molekularem Sauerstoff im Reaktionsgasausgangsgemisch 1 ist erfindungsgemäß normalerweise so bemessen (wie bereits erwähnt), dass das molare Verhältnis V₁ von im Reaktionsgasausgangsgemisch 1 enthaltenem O₂ zu im Reaktionsgasausgangsgemisch 1 enthaltenem C₃H₆ ≥ 1 beträgt. Üblicherweise beträgt V₁ beim erfindungsgemäßen Verfahren ≥ 1 und ≤ 3, meist ≥ 1,3 und ≤ 2,5, oft ≥ 1,5 bis ≤ 2,3. Die Menge an molekularem Sauerstoff im Reaktionsgasausgangsgemisch 2 (das Reaktionsgasgemisch mit dem das Katalysatorfestbett der zweiten Reaktionsstufe beschickt wird) wird, wie bereits erwähnt, normalerweise so bemessen, dass das molare Verhältnis von im Reaktionsgasausgangsgemisch 2 enthaltenem O₂ zu im Reaktionsgasausgangsgemisch 2 enthaltenem Acrolein ≥ 0,5 bis ≤ 3, bzw. ≤ 2, häufig ≥ 0,75 bis ≤ 1,5 beträgt.

Auch kann das Reaktionsgasausgangsgemisch 1 ≥ 0,01, oder ≥ 0,1, oder ≥ 0,5, oder ≥ 2 Vol.-% an CO₂ enthalten. Meist wird der vorgenannte CO₂-Gehalt ≤ 25 Vol.-% betragen.

Insbesondere dann, wenn als Quelle für den molekularen Sauerstoff beim erfindungsgemäßen Verfahren Luft verwendet wird, wird das Reaktionsgasausgangsgemisch 1 als weiteres inertes Verdünnungsgas molekularen Stickstoff enthalten. Grundsätzlich kann das Reaktionsgasausgangsgemisch 1 beim erfindungsgemäßen Verfahrene ≥1 Vol.-%, oder ≥5 Vol.-%, oder ≥ 10 Vol.-%, oder ≥ 20 Vol.-%, oder ≥ 30 Vol.-%, oder ≥ 40 Vol.-% an molekularem Stickstoff enthalten. In der Regel wird der Gehalt des Reaktionsgasausgangsgemischs 1 an molekularem Stickstoff jedoch bei Werten ≤ 80 mol-%, oder ≤ 70 mol-%, oder ≤ 60 mol-% liegen.

Auch kann das Reaktionsgasausgangsgemisch 1 Propan als inertes Verdünnungsgas enthalten. Dieser Propangehalt des Reaktionsgasausgangsgemischs 1 kann bis zu 70 Vol.-% (z. B. 5 bis 70 Vol.-%), bzw. bis zu 60 Vol.-%, oder bis 50 Vol.-%, oder bis zu 40 Vol.-%, oder bis 30 Vol.-%, oder bis 20 Vol.-%, oder bis zu 10 Vol.-% betragen. Häufig liegt dieser Propangehalt bei ≥ 0,5 bzw. ≥ 1 Vol.-%. Er kann aber auch bei Werten von ≥ 0,01 Vol.-%, oder ≥ 0,02 Vol.-%, oder ≥ 0,03 Vol.-% liegen. In der Regel enthält das Reaktionsgasausgangsgemisch 1 ≤ 10 Vol.-%, vielfach ≤ 5 Vol.-% an Propan.

Dieses Propan kann beim erfindungsgemäßen Verfahren z. B. bewusst als dem Reaktionsgasausgangsgemisch 1 separat zuzuführendes inertes Verdünnungsgas zugesetzt werden.

Selbstverständlich kann das Propan aber auch dadurch Bestandteil das Reaktionsgasausgangsgemisch 1 werden, dass als Propylenquelle für selbiges eine partielle Dehydrierung oder Oxidehydrierung von Propan fungiert (in der Regel werden diese heterogen katalysiert erfolgen). D. h., das im Reaktionsgasausgangsgemisch 1 enthaltene Propylen kann dem Reaktionsgasausgangsgemisch 1 wenigstens teilweise unter Begleitung von nicht umgesetztem Propan aus einer partiellen Dehydrierung (z. B. homogen und/oder heterogen katalysiert, im Beisein und/oder unter Ausschluss von molekularem Sauerstoff) zugeführt werden.

Das erfindungsgemäße Verfahren umfasst insbesondere auch solche Ausführungsformen, bei denen das Reaktionsgasausgangsgemisch 1 > 0 bis 35 Vol.-%, häufig 1 bis 25 Vol.-%, oder 5 bis 15 Vol.-%, bzw. bis 10 Vol.-% H₂O enthält.

Typische Reaktionsgasausgangsgemische 1 sind z. B. solche, die enthalten:

| | | |
|---|---|---|
| | 5 oder 6 bis 11 Vol.-% | Propen, |
| | 2 oder 6 bis 12 Vol.-% | Wasser, |
| > 0, häufig ≥ 0,5 oder ≥ | 1 bis 10 Vol.-% | Propan, |
| | ≥ 0 bis 5 Vol.-% | von Propen, Propan, Wasser, |
| | | Sauerstoff und Stickstoff verschiedene Bestandteile, |
| | soviel molekularen Sauerstoff, dass V₁ 1 bis 3 beträgt, und als Restmenge bis zu 100 Vol.-% Gesamtmenge molekularen Stickstoff. | |

Erfindungsgemäße Reaktionsgasausgangsgemische 1 können auch enthalten:

| | |
|---|---|
| 6 bis 9 Vol.-% | Propylen, |
| 8 bis 18 Vol.-% | molekularer Sauerstoff, |
| 6 bis 30 bzw. bis 35 Vol.-% | Propan und |
| 32 bis 72 Vol.-% | molekularer Stickstoff. |

Erfindungsgemäße Reaktionsgasausgangsgemische 2 können z. B. enthalten:

| | |
|---|---|
| 4,5 bis 8 Vol.-% | Acrolein, |
| 2,25 bis 9 Vol.-% | molekularer Sauerstoff, |
| 6 bis 30 bzw. bis 35 Vol.-% | Propan, |
| 32 bis 72 Vol.-% | molekularer Stickstoff, |
| 5 bis 30 Vol.-% | Wasserdampf. |

Erfindungsgemäße Reaktionsgasausgangsgemische 1 können aber auch bis zu 20 Vol.-% H₂ enthalten.

D.h., Reaktionsgasausgangsgemische 1 des erfindungsgemäßen Verfahrens können auch enthalten:

| | |
|---|---|
| 4 bis 25 Vol.-% | Propylen, |
| 6 bis 70 Vol.-% | Propan, |
| 5 bis 60 Vol.-% | H₂O, |
| 8 bis 65 Vol.-% | O₂ und |
| 0,3 bis 20 Vol.-% | H₂. |

Das erfindungsgemäße Verfahren ist aber auch dann günstig, wenn das Reaktionsgasausgangsgemisch 1 0,1 bis 30 Vol.-% CO₂ enthält.

Erfindungsgemäß mögliche Reaktionsgasausgangsgemische 2 können auch enthalten:

| | |
|---|---|
| 3 bis 25 Vol.-% | Acrolein, |
| 5 bis 65 Vol.-% | molekularer Sauerstoff, |
| 6 bis 70 Vol.-% | Propan, |
| 0,3 bis 20 Vol.-% | molekularer Wasserstoff und |
| 8 bis 65 Vol.-% | Wasserdampf. |

Erfindungswesentlich ist, dass für alle vorgenannten Konstellationen das erfindungsgemäße Verfahren jeweils für beide Stufen sowohl dann angewendet werden kann, wenn die beiden Stufen voneinander unabhängig betrieben werden, als auch wenn sie wie vorstehend dargestellt in Hintereinanderschaltung betrieben werden. Es ist aber auch dann erfolgreich, wenn beide Schritte, wie in der DE-A 101 21 592 beschrieben, in einem Reaktor an einer Beschickung vollzogen werden.

Dabei kann sich der Teilkatalysatorfestbettwechsel (wie ganz generell bei den erfindungsgemäßen Verfahren dieser Schrift) in allen Fällen in Strömungsrichtung (des Reaktionsgasgemischs) auf bis zu 80 %, oder nur auf bis zu 70 %, oder nur auf bis zu 60 %, oder nur auf bis zu 50 %, oder nur auf bis zu 40 %, oder nur auf bis zu 30 %, oder bevorzugt auf bis zu 25 %, besonders bevorzugt auf 30 bis 50 % und ganz besonders bevorzugt auf 35 bis 45 % der Schüttlänge des jeweiligen Katalysatorfestbettes erstrecken (eine zu 100 % aus Inertmaterial bestehende Deckbeschickung (die Erstbeschickung aus der Strömungssicht) wird dabei nicht zum Katalysatorfestbett zugehörig betrachtet; aus Gründen der Zweckmäßigkeit würde diese Deckbeschickung aber mit ausgetauscht; in entsprechender Weise würde auch eine zu 100 % aus Inertmaterial bestehende Abschlußbeschickung (die Endbeschickung aus der Strömungssicht) nicht zum Katalysatorfestbett zugehörig betrachtet; eine zu 100 % aus Inertmaterial bestehende Zwischenbeschickung würde jedoch zum Katalysatorfestbett zugehörig betrachtet). Zweckmäßigerweise beträgt der vorgenannten Prozentsatz für einen Teilkatalysatorwechsel häufig nicht weniger als 10 % bzw. 20 %.

Abschließend sei nochmals erwähnt, dass insbesondere ein Teil des Beschickungsgasgemisches der ersten Stufe ("Propylen → Acrolein ") sogenanntes Kreisgas sein kann. Hierbei handelt es sich um Gas, das nach der Produktabtrennung (Acrylsäureabtrennung) vom Produktgasgemisch der zweiten Stufe verbleibt und bei einer Hintereinanderschaltung der beiden Stufen in der Regel zum Teil als inertes Verdünnungsgas zum Beschicken der ersten und/oder zweiten Stufe rückgeführt wird.

Eine typische Kreisgaszusammensetzung lautet:

| | |
|---|---|
| 0 - 0,1 Vol.-% | sonstige, z. B. Diphenyl, Diphenylether und/oder Dimethylphthalat, |
| 0 - 0,1 Vol.-% | Acrylsäure, |
| 0 - 0,1 Vol.-% | Acrolein, |
| 3 - 5 Vol.-% | Sauerstoff, |
| 1 - 5 Vol.-% | Wasserdampf, |
| 0 - 3 Vol.-% | Kohlenmonoxid, |
| 0 - 8 Vol.-% | Kohlendioxid, |
| 0 - 2 Vol.-% | Propan, |
| 0,1 - 0,5 Vol.-% | Propylen, |
| 85 - 95 Vol.-% | Stickstoff. |

Dabei kann die Acrylsäureabtrennung z. B. wie in der EP-A 982 287, der EP-A 982 289, der DE-A 199 24 532, der DE-A 101 15 277, der DE-A 196 06 877, der DE-A 197 40 252, der DE-A 196 27 847, der DE-A 100 53 086, der EP-A 982 288 und der DE-A 196 27 847 beschrieben abgetrennt werden.

Prinzipiell kann der Teilkatalysatorfestbettwechsel zu jedem Zeitpunkt, d. h. z. B. nach einjähriger, zweijähriger, dreijähriger oder mehrjähriger Betriebsdauer durchgeführt werden. In der Regel wird man ihn gemäß Wirtschaftlichkeitsüberlegungen durchführen.

Abschließend sei erwähnt, dass sich der erfindungsgemäße Teilkatalysatorfestbettwechsel in der Regel auch vorteilhaft auf den Druckverlust beim Durchgang des Reaktionsgasgemischs durch die Katalysatorbeschickung auswirkt.

Außerdem sei nochmals erwähnt, dass die Wärmeaustauschmittel (Wärmeträger, Salzschmelzen) bevorzugt in solchen Mengen durch die relevanten Vielkontaktrohr-Festbettreaktoren geführt werden, dass der Unterschied zwischen ihrer Eingangs- und ihrer Ausgangstemperatur ≤ 5 °C beträgt.

### Beispiel und Vergleichsbeispiel

### 1. Allgemeine Beschreibung der ersten Reaktionsstufe

| | | |
|---|---|---|
| Verwendeter Wärmeträger: | Salzschmelze aus 60 Gew.-% Kaliumnitrat und 40 Gew.-% Natriumnitrit. Wird zum Reaktions- gasgemisch im Gegenstrom geführt. Sie wurde bei frischer Beschickung des Kontaktrohres mit Katalysatorfestbett mit einer Temperatur von 320 °C zugeführt und mit einer Temperatur von 322 °C abgeführt. | |
| | | |
| Material des in einem Viel-Kontaktrohr-Festbettreaktor befindlichen Kontaktrohrs: | ferritischer Stahl | |
| | | |
| Zusammensetzung des Kontaktrohres: | 3200 mm Länge 26 mm Innendurchmesser 31 mm Außendurchmesser (Wandstärke 2,5 mm) | |
| | | |
| Zusammensetzung des Reak tionsgasausgangsgemischs 1: | 5,4 Vol.-% 10,5 Vol.-% 1,2 Vol.-% 81,3 Vol.-% 1,6 Vol.-% | Propylen, molekularer Sauerstoff, COₓ, N₂, und H₂O. |
| | | |
| Propylenbelastung der Kata lysatorbeschickung: | 110 Nl/l·h | |
| | | |
| Frische Beschickung des Kon taktrohres (in Strömungsrichtung des Reaktionsgasgemischs) | Zone A: 50 cm Vorschüttung aus Steatit-Ringen der Geometrie 7 mm x 7 mm x 4 mm (Außendurchmesser x Länge x Innendurchmesser) Zone B: 100 cm Katalysatorbeschickung mit einem homogenen Gemisch aus 30 Gew.-% an Steatit-Ringen (Steatit C 220 der Fa. CeramTec) der Geomtrie 5 mm x 3 mm x 2 mm (Außendurchmesser x Länge x Innendurchmesser) und 70 Gew.-% Vollkatalysator aus Zone C. Zone C: 170 cm Katalysatorbeschickung mit ringförmigem (5 mm x 3 mm x 2 mm = Außendurchmesser x Länge x Innendurchmesser) Vollkatalysator ge- mäß Beispiel 1 der DE-A 100 46 957 | |

### II. Zwischenkühlung und Sekundärgaszugabe

Das Produktgasgemisch der ersten Reaktionsstufe wurde im wesentlichen ohne Acroleinverlust in einem Rohrbündelwärmetauscher durch indirekten Wärmeaustausch mit einer Salzeschmelze aus 60 Gew.-% Kaliumnitrat und 40 Gew.-% Natriumnitrit auf 250 °C abgekühlt. Anschließend wurde eine Temperatur von 140 °C aufweisende komprimierte Luft in einer solchen Menge zugegeben, dass das molare Verhältnis von O₂ : Acrolein im resultierenden Gemisch ca. 1,28 betrug. Dieses Gemisch wurde mit einer Temperatur von 220 °C der zweiten Reaktionsstufe zugeführt.

### III. Allgemeine Beschreibung der zweiten Reaktionsstufe

Das Kontaktrohr entsprach jenem in der ersten Reaktionsstufe. Salzschmelze (gleiche Zusammensetzung wie in erster Reaktionsstufe) und Reaktionsgasgemisch wurden im Gegenstrom geführt. Die Salzschmelze wurde bei frischer Beschickung des Kontaktrohres mit Katalysatorfestbett mit einer Temperatur von 260 °C zugeführt und mit einer Temperatur von 262 °C abgeführt.

| | |
|---|---|
| Die frische Beschickung des Kontaktrohres war (in Strömungsrichtung des Reaktionsgasgemischs) | Zone A: 20 cm Vorschüttung aus Steatit-Ringen (Steatit C 220 der Fa. CeramTec) der Geometrie 7 mm x 7 mm x 4 mm (Außendurchmesser x Länge x Innendurchmesser). Zone B: 100 cm Katalysatorbeschickung (alternativ: 120 cm) mit einem homogenen Gemisch aus 30 (alternativ: 35) Gew.-% an Steatit-Ringen (Steatit C 220 der Fa. CeramTec) der Geometrie 7 mm x 3 mm x 4 mm (Außendurchmesser x Länge x In- nendurchmesser) und 70 (alternativ: 65) Gew.-% Schalenkatalysator aus Zone C. Zone C: 200 (alternativ: 180) cm Katalysatorbeschickung mit ringfömrigem (ca. 7 mm x 3 mm x 4 mm = Außendurchmesser x Länge x Innendurchmes- ser) Schalenkatalysator gemäß Herstellungsbei- spiel 5 der DE-A 100 46 928 |

### IV. Ergebnisse (die Selektivität der Acrylsäurebildung blieb weitgehend konstant)

### A) Beispiel

Die Analyse des Produktgasgemischs der zweiten Reaktionsstufe ergab folgende Ergebnisse:

Der Umsatz des in der ersten Reaktionsstufe gebildeten Acrolein betrug mit in die zweite Reaktionsstufe frisch eingebrachtem Katalysatorfestbett (nach abgeschlossener Formierung desselben) bei einer Eintrittstemperatur T^{ein} der Salzschmelze in die zweite Stufe von 260 °C 99,3 mol-% bei einer Selektivität der Acrylsäurebildung von 88,9 mol-% (diese Angaben sind ebenso wie die nachfolgenden Angaben auf einfachen Durchgang bezogen).

Mit zunehmender Betriebsdauer fiel der Acroleinumsatz in der zweiten Reaktionsstufe. Durch sukzessives Erhöhen der Eintrittstemperatur der Salzschmelze in die zweite Reaktionsstufe konnte dieser Aktivitätsverlust ausgeglichen werden (die Deaktivierungsrate lag stabil bei 8 °C/Jahr).

Als T^{ein} 283 °C erreicht war, lag ΔT^{HB}ᵥ bei 33 °C. Nun wurde das Verfahren unterbrochen und in der zweiten Reaktionsstufe die gesamte Zone A sowie die gesamte Zone B abgesaugt und durch eine frische Zone A sowie eine frische Zone B ersetzt, wobei die frische Zone B jedoch 50 Gew.-% an den Steatit-Ringen der Geometrie 7 mm x 3 mm x 4 mm und nur noch 50 Gew.-% an frischem Schalenkatalysator gemäß Herstellungsbeispiel 5 der DE-A 100 46 928 aufwies.

Mit einem T^{ein} von 275 °C konnte anschließend bei einer Deaktivierungsrate von 12 °C/Jahr und einem ΔT^{HB}ₙ von 32 °C das Verfahren ansonsten unverändert bei 99,3 mol-% Acroleinumsatz fortgesetzt werden.

### B) Vergleichsbeispiel

Es wurde wie im Beispiel verfahren. Die abgesaugte Zone B wurde jedoch durch eine frische Zone B ersetzt, die wie die ursprüngliche Zone B nur 30 Gew.-% an den Steatit-Ringen der Geometrie 7 mm x 3 mm x 4 mm und 70 Gew.-% an frischem Schalenkatalysator gemäß Herstellungsbeispiel 5 der DE-A 100 46 928 aufwies.

Zwar konnte das Verfahren anschließend mit einem Acroleinumsatz von 99,3 mol-% bei einem T^{ein} von nur 270 °C fortgesetzt werden, doch lag ΔT^{HB}ₙ bei 48 °C und die Deaktivierungsrate betrug 20 °C/Jahr.

US Provisional Patent Application No. 60/756,207, eingereicht am 05.01.2006, ist in die vorliegende Patentanmeldung durch Literaturhinweis eingefügt.

Im Hinblick auf die obengenannten Lehren sind zahlreiche Änderungen und Abweichungen von der vorliegenden Erfindung möglich.

Man kann deshalb davon ausgehen, dass die Erfindung, im Rahmen der beigefügten Ansprüche, anders als hierin spezifisch beschrieben, ausgeführt werden kann.

## Patentansprüche

1. Verfahren der heterogen katalysierten Gasphasen-Partialoxidation wenigstens einer organischen Ausgangsverbindung mit molekularem Sauerstoff an einem in einen Reaktionsraum frisch eingebrachten Katalysatorfestbett, bei dem man zum Zweck der Partialoxidation ein die wenigstens eine organische Ausgangsverbindung und den molekularen Sauerstoff enthaltendes Reaktionsgasgemisch durch das Katalysatorfestbett führt sowie durch indirekten Wärmeaustausch mit einem außerhalb des Reaktionsraums geführten fluiden Wärmeträger Reaktionswärme abführt und dann, wenn mit zunehmender Betriebsdauer eine zunehmende Minderung der Qualität des Katalysatorfestbetts einhergeht, zur Rückgewinnung der Qualität des Katalysatorfestbetts nicht das gesamte Katalysatorfestbett sondern nur einen Teil desselben durch ein Ersatzkatalysatorteilfestbett ersetzt, **dadurch gekennzeichnet, dass** die volumenspezifische Aktivität des Ersatzkatalysatorteilfestbetts geringer ist, als die volumenspezifische Aktivität des ersetzten Katalysatorfestbettteils in seinem frisch eingebrachten Zustand.

2. Verfahren nach Anspruch 1, **dadurch gekennzeichnet, dass** die Gasphasen-Partialoxidation diejenige von Propylen zu Acrolein und/oder Acrylsäure, oder diejenige von iso-Buten zu Methacrolein und/oder Methacrylsäure, oder diejenige von Acrolein zu Acrylsäure, oder diejenige von Methacrolein zu Methacrylsäure, oder diejenige von Propan zu Acrylsäure, oder diejenige von iso-Butan zu Methacrylsäure ist.

3. Verfahren nach Anspruch 1, **dadurch gekennzeichnet, dass** es sich bei der wenigstens einen organischen Ausgangsverbindung um wenigstens eine organische Ausgangsverbindung aus der Gruppe umfassend Propylen, Acrolein, 1-Buten, 2-Buten, Ethan, Benzol, m-Xylol, p-Xylol, iso-Butan, iso-Buten, tert.-Butanol, iso-Butyraldehyd, Methylether des tert.-Butanols, o-Xylol, Naphtalin, Butadien, Ethylen, Propan oder Methacrolein handelt.

4. Verfahren nach einem der Ansprüche 1 bis 3, **dadurch gekennzeichnet, dass** die Gasphasen-Partialoxidation die zweite Stufe einer zweistufigen Gasphasen-Partialoxidation ist.

5. Verfahren nach Anspruch 4, **dadurch gekennzeichnet, dass** die Gasphasen-Partialoxidation die Partialoxidation von Acrolein zu Acrylsäure in einer zweistufigen Gasphasen-Partialoxidation von Propylen zu Acrylsäure ist.

6. Verfahren nach einem der Ansprüche 1 bis 5, **dadurch gekennzeichnet, dass** man die volumenspezifische Aktivität des Ersatzkatalysatorteilfestbetts so bemisst, dass, bezogen auf gleichen Umsatz der organischen Ausgangsverbindung bei Einmaldurchgang des Reaktionsgasgemischs durch das Katalysatorfestbett sowie gleiche Zusammensetzung des Reaktionsgasgemischs und gleiche Belastung des Katalysatorfestbetts mit Reaktionsgasgemisch, die Differenz dΔT zwischen der Heißpunktausdehnung ΔT^{HB}ₙ des Katalysatorfestbetts nach dem durchgeführten Ersatz durch das Ersatzkatalysatorteilfestbett und der Heißpunktausdehnung ΔT^{HB}ᵥ vor der Durchführung des Ersatzes durch das Ersatzkatalysatorteilfestbett, gebildet als dΔT = ΔT^{HB}ₙ-ΔT^{HB}ᵥ, ≤ 30 °C beträgt.

7. Verfahren nach Anspruch 6, **dadurch gekennzeichnet, dass** dΔT -15 bis +10 °C beträgt.

8. Verfahren nach Anspruch 6, **dadurch gekennzeichnet, dass** dΔT - 10 bis 0 °C beträgt.

9. Verfahren nach einem der Ansprüche 1 bis 8, **dadurch gekennzeichnet, dass** der Reaktionsraum das Innere eines Reaktionsrohres ist.

10. Verfahren nach Anspruch 9, **dadurch gekennzeichnet, dass** sich das Reaktionsrohr in einem Rohrbündelreaktor befindet.

11. Verfahren nach einem der Ansprüche 1 bis 10, **dadurch gekennzeichnet, dass** sich der durch das Ersatzkatalysatorteilfestbett ersetzte Teil des Katalysatorfestbetts in Strömungsrichtung des Reaktionsgasgemischs auf bis zu 80% der Schüttlänge des Katalysatorfestbetts erstreckt.

## Claims

1. A process for heterogeneously catalyzed gas phase partial oxidation of at least one organic starting compound with molecular oxygen over a fixed catalyst bed freshly installed into a reaction chamber, in which, for the purpose of the partial oxidation, a reaction gas mixture comprising the at least one organic starting compound and the molecular oxygen is conducted through the fixed catalyst bed and heat of reaction is removed by indirect heat exchange with a fluid heat carrier conducted outside the reaction chamber, and, when increasing operating time is accompanied by an increasing reduction in the quality of the fixed catalyst bed, the quality of the fixed catalyst bed is recovered by replacing not the entire fixed catalyst bed but only a portion thereof by a replacement fixed catalyst bed part, wherein the volume-specific activity of the replacement fixed catalyst bed part is lower than the volume-specific activity of the replaced fixed catalyst bed part in its freshly installed state.

2. The process according to claim 1, wherein the gas phase partial oxidation is that of propylene to acrolein and/or acrylic acid, or that of isobutene to methacrolein and/or methacrylic acid, or that of acrolein to acrylic acid, or that of methacrolein to methacrylic acid, or that of propane to acrylic acid, or that of isobutane to methacrylic acid.

3. The process according to claim 1, wherein the at least one organic starting compound is at least one organic starting compound from the group comprising propylene, acrolein, 1-butene, 2-butene, ethane, benzene, m-xylene, p-xylene, isobutane, isobutene, tert-butanol, isobutyraldehyde, methyl ether of tert-butanol, o-xylene, naphthalene, butadiene, ethylene, propane or methacrolein.

4. The process according to any of claims 1 to 3, wherein the gas phase partial oxidation is the second stage of a two-stage gas phase partial oxidation.

5. The process according to claim 4, wherein the gas phase partial oxidation is the partial oxidation of acrolein to acrylic acid in a two-stage gas phase partial oxidation of propylene to acrylic acid.

6. The process according to any of claims 1 to 5, wherein the volume-specific activity of the replacement fixed catalyst bed part is such that, based on the same conversion of the organic starting compound in single pass of the reaction gas mixture through the fixed catalyst bed and the same composition of the reaction gas mixture and the same loading of the fixed catalyst bed with reaction gas mixture, the difference dΔT between the hotspot expansion ΔT^{HB}ₙ of the fixed catalyst bed after the replacement by the replacement fixed catalyst bed part has been carried out and the hotspot expansion ΔT^{HB}ᵥ before the replacement by the replacement fixed catalyst bed part has been carried out, formed as dΔT = ΔT^{HB}ₙ-ΔT^{HB}ᵥ, is ≤ 30°C.

7. The process according to claim 6, wherein dΔT is from -15 to +10°C.

8. The process according to claim 6, wherein dΔT is from -10 to 0°C.

9. The process according to any of claims 1 to 8, wherein the reaction chamber is the interior of a reaction tube.

10. The process according to claim 9, wherein the reaction tube is disposed in a tube bundle reactor.

11. The process according to any of claims 1 to 10, wherein the part of the fixed catalyst bed replaced by the replacement fixed catalyst bed part extends in flow direction of the reaction gas mixture to up to 80% of the bed length of the fixed catalyst bed.

## Revendications

1. Procédé d'oxydation partielle en phase gazeuse sous catalyse hétérogène d'au moins un composé de départ organique avec de l'oxygène moléculaire sur un lit solide catalytique fraîchement introduit dans une chambre de réaction, selon lequel, dans le but de l'oxydation partielle, un mélange gazeux réactionnel contenant au moins un composé de départ organique et l'oxygène moléculaire est passé au travers du lit catalytique solide et la chaleur de réaction est dissipée par échange de chaleur indirect avec un fluide caloporteur mis en circulation à l'extérieur de la chambre de réaction, puis lorsque la durée d'exploitation croissante s'accompagne d'une diminution croissante de la qualité du lit solide catalytique, pour récupérer la qualité du lit solide catalytique, seule une partie du lit solide catalytique, et non la totalité, est remplacée par un lit solide catalytique partiel de remplacement, **caractérisé en ce que** l'activité spécifique au volume du lit solide catalytique partiel de remplacement est plus faible que l'activité spécifique au volume de la partie du lit solide catalytique remplacée dans son état fraîchement introduit.

2. Procédé selon la revendication 1, **caractérisé en ce que** l'oxydation partielle en phase gazeuse est celle de propylène en acroléine et/ou en acide acrylique, ou celle d'iso-butène en méthacroléine et/ou en acide méthacrylique, ou celle d'acroléine en acide acrylique, ou celle de méthacroléine en acide méthacrylique, ou celle de propane en acide acrylique, ou celle d'isobutane en acide méthacrylique.

3. Procédé selon la revendication 1, **caractérisé en ce que** le composé de départ organique est au moins un composé de départ organique du groupe comprenant le propylène, l'acroléine, le 1-butène, le 2-butène, l'éthane, le benzène, le m-xylène, le p-xylène, l'isobutane, l'iso-butène, le tert.-butanol, l'isobutyraldéhyde, l'éther méthylique du tert.-butanol, le o-xylène, la naphtaline, le butadiène, l'éthylène, le propane ou la méthacroléine.

4. Procédé selon l'une quelconque des revendications 1 à 3, **caractérisé en ce que** l'oxydation partielle en phase gazeuse est la seconde étape d'une oxydation partielle en phase gazeuse à deux étapes.

5. Procédé selon la revendication 4, **caractérisé en ce que** l'oxydation partielle en phase gazeuse est l'oxydation partielle d'acroléine en acide acrylique dans le cadre d'une oxydation partielle en phase gazeuse à deux étapes de propylène en acide acrylique.

6. Procédé selon l'une quelconque des revendications 1 à 5, **caractérisé en ce que** l'activité spécifique au volume du lit solide catalytique partiel de remplacement est déterminée de manière à ce que, pour une conversion identique du composé de départ organique par un passage unique du mélange gazeux réactionnel au travers du lit solide catalytique, et avec une composition identique du mélange gazeux réactionnel et une charge identique du lit solide catalytique en mélange gazeux réactionnel, la différence dΔT entre la dilatation du point chaud ΔT^{HB}ₙ du lit solide catalytique après la réalisation du remplacement par le lit solide catalytique partiel de remplacement et la dilatation du point chaud ΔT^{HB}ᵥ avant la réalisation du remplacement par le lit solide catalytique partiel de remplacement, représentée par dΔT = ΔT^{HB}ₙ - ΔT^{HB}ᵥ , est inférieure ou égale à 30 °C.

7. Procédé selon la revendication 6, **caractérisé en ce que** dΔT est de -15 à +10 °C.

8. Procédé selon la revendication 6, **caractérisé en ce que** dΔT est de -10 à 0 °C.

9. Procédé selon l'une quelconque des revendications 1 à 8, **caractérisé en ce que** la chambre de réaction est l'intérieur d'un tube de réaction.

10. Procédé selon la revendication 9, **caractérisé en ce que** le tube de réaction se trouve dans un réacteur à faisceau de tubes.

11. Procédé selon l'une quelconque des revendications 1 à 10, **caractérisé en ce que** la partie du lit solide catalytique remplacée par le lit solide catalytique partiel de remplacement atteint jusqu'à 80 % de la longueur de garnissage du lit solide catalytique dans la direction de l'écoulement du mélange gazeux réactionnel.
